# FASCICULE DE BREVET EUROPEEN

(11) **EP 0 906 127 B1**
(45) Date de publication et mention de la délivrance du brevet: **17.04.2002**
(21) Numéro de dépôt: 97928317.3
(22) Date de dépôt: 10.06.1997
(51) Int. Cl.: A61L 15/44, A61K 31/575, A61P 5/24

(54) **NOUVEAUX DISPOSITIFS DESTINES A L'ADMINISTRATION TRANSDERMIQUE DE LA TRIMEGESTONE**
NEUE VORRICHTUNGEN ZUR TRANSDERMALEN VERABREICHUNG VON TRIMEGESTONE
NOVEL DEVICES FOR TRANSDERMAL ADMINISTERING OF TRIMEGESTONE

(30) Priorité: 11.06.1996 FR 9607208
(43) Date de publication de la demande: 07.04.1999
(73) Titulaire: Aventis Pharma S.A., 92160 Antony (FR)
(72) Inventeur: DUBOIS, Jean-Luc, F-75019 Paris (FR)
(86) Numéro de dépôt international: FR9701023
(87) Numéro de publication internationale: WO9747333

(56) Documents cités:
- EP-A- 0 007 823
- EP-A- 0 328 806
- EP-A- 0 483 105
- WO-A-87/07138
- DE-A- 4 309 830
- DE-A- 4 405 899
- US-A- 5 186 939
- KENJU SUGIBAYASHI ET AL: "POLYMERS FOR TRANSDERMAL DRUG DELIVERY SYSTEMS" JOURNAL OF CONTROLLED RELEASE, vol. 29, no. 1/02, 1 février 1994, pages 177-185, XP000433662

## Description

La présente invention concerne de nouveaux dispositifs destinés à une administration par voie transdermique de la trimegestone et leur procédé de préparation.

Les systèmes transdermiques délivrant un progestomimétique présentent un grand intérêt en thérapeutique.

En effet, les stéroïdes progestomimétiques sont des principes actifs qui doivent être administrés sur de longues périodes de temps.

Des esters retard de ces stéroides ou des formes galéniques à libération contrôlée telles que les implants ou les microsphères sont utilisés en thérapeutique mais présentent le plus souvent l'inconvénient de ne pas être d'une grande facilité d'emploi. De plus, ils ne permettent pas d'interrompre le traitement très rapidement en cas de nécessité. Un système transdermique a l'avantage de pouvoir être utilisé pour un traitement à long terme tout en limitant les surdoses. De plus la voie transdermique permet d'éviter les effets indésirables liés à la surcharge au niveau hépatique.

La demanderesse a ainsi été amenée à étudier une nouvelle composition pharmaceutique administrable par voie transdermique renfermant un progestomimétique connu sous le nom de Trimegestone (le 17alpha-méthyl-17béta-(2-hydroxy-1-oxo-propyl)-estra-4,9-dièn-3-one (21S)) :
1 - qui permette de contenir la Trimegestone dans de bonnes conditions de stabilité, notamment lors du stockage,
2 - qui permette d'administrer la Trimegestone avec un flux transcutané compris entre 0,1 et 3 µg·cm⁻²·h⁻¹,
3 - qui permette de délivrer la dose nécessaire de principe actif tout en gardant une dimension (surface) plus acceptable que celle des patchs de l'art antérieur,
4 - dont le procédé de fabrication est simple et peu coûteux
5 - qui permette une adhésion compatible avec la durée nécessaire notamment à un traitement hormonal substitutif de la ménopause, soit de 3 à 7 jours,
6 - qui présente un grammage adéquat aux critères de flux et d'adhésion.

L'invention a donc pour objet une matrice polymère adhésive appliquée à un dispositif destiné à l'administration transdermique d'un progestomimétique caractérisée en ce que cette matrice est constituée d'une ou des couches successives suivantes :
- éventuellement une couche (1), dite d'ancrage, constituée d'un polymère silicone,
- une couche (2), constituée d'un polymère silicone chargé en Trimegestone et/ou en un ou plusieurs dérivés pharmaceutiquement acceptables, et éventuellement en plastifiant,
- éventuellement une couche (3), dite d'adhésion, constituée d'un polymère silicone.

La Trimegestone est un puissant progestomimétique décrit dans le brevet Européen EP-0007823.

On entend par dérivés pharmaceutiquement acceptables, les esters en position 21 de la Trimegestone, le reste du groupement ester renfermant de 1 à 12 atomes de carbone. Il s'agit notamment du dérivé 17béta-(2-acétoxy-1-oxo propyl) estra-4,9-dièn-3-one (21S). Il peut s'agir également des groupements 2-acyloxy suivants : isopropionyloxy, propionyloxy, butyryloxy, isobutyryloxy, valéryloxy, isovaléryloxy, oxalyloxy, succinyloxy, pivaloyloxy, undécanoyloxy, benzoyloxy. Ces esters sont notamment préparés par action d'acides ou d'anhydrides carboxyliques aliphatiques ou aromatiques renfermant de 1 à 12 atomes de carbone sur la Trimegestone.

Les esters de la Trimegestone, dont le reste ester renferme 1 atome de carbone ou de 3 à 12 atomes de carbone, sont également objet de la présente invention.

Parmi les nombreux polymères connus de l'homme du métier, susceptibles de contenir un progestomimétique tel que la Trimegestone, la demanderesse a déterminé que seul un polymère silicone permet de répondre aux conditions de stabilité, de flux et d'adhésion requises.

On entend par polymère silicone, un réseau de chaînes polydiméthylsiloxane (P.D.M.S.).

Deux types de polymères peuvent être utilisés, l'un au pouvoir adhésif instantané fort et notamment le BIO-PSA® 7-4301, l'autre au pouvoir adhésif instantané moyen et notamment le BIO-PSA® 7-3045, 7-4201 ou 7-4202. (Société Dow Corning Health Care Centre Europe). Le polymère silicone se présente sous la forme d'une solution limpide dans un solvant organique volatil à basse température tel que l'hexane, l'heptane, l'acétate d'éthyle ou le tétrahydrofuranne.

Ces polymères sont amino-résistants, ils ne présentent pas de groupements silanol acides.

Le pouvoir adhésif est caractérisé par la force de pelage et la force d'adhésion : ce pouvoir adhésif augmente lorsque l'on passe du grade pouvoir adhésif instantané faible à pouvoir adhésif instantané moyen puis pouvoir adhésif instantané fort. Le paramètre permettant de moduler cette caractéristique physique est le ratio polymère/résine de silicone. Par pouvoir adhésif moyen on entend un rapport 40/60. Par pouvoir adhésif fort, on entend un rapport 45/55.

La quantité de Trimegestone incorporée dans une matrice polymère telle que définie précédemment est de préférence comprise entre 1 % p/p et 10 % p/p. Ce pourcentage correspond à la quantité de principe actif exprimé par rapport au poids sec, après évaporation du solvant, du mélange. La Trimegestone se trouve alors à l'état de suspension au sein de la matrice silicone.

Les plastifiants ont pour but d'augmenter la valeur de l'adhésion instantanée. On utilise les huiles telles que l'huile de silicone ou les Cétiols tels que le Cétiol® S (dioctylcyclohexane). Il s'agira tout particulièrement d'huile de silicone haute viscosité. Il s'agit de diméthylsiloxane polymérisé et tout particulièrement d'huile de silicone 7-9120 de la société DOW Corning (12000 cSt). La quantité de plastifiant peut s'échelonner entre 1 et 10 % p/p. De préférence, on choisira de 1 à 3 % p/p de plastifiant.

Cette matrice silicone présente un certain nombre d'avantages :
- Absence de promoteur d'absorption,
- La Trimegestone est bien stabilisée chimiquement dans le polymère silicone,
- Les niveaux de flux et d'adhésion sont compatibles avec un traitement thérapeutique,
- Le système matriciel a l'avantage par rapport aux systèmes avec réservoir, de ne pas présenter de risque de rupture d'une membrane ce qui peut entraîner une surdose du principe actif.
- La Trimegestone n'est pas affectée lors de la mise en oeuvre du procédé de fabrication alors qu'elle est instable à l'humidité et à la température.

Les patchs renfermant la Trimegestone tels que décrits précédemment permettent ainsi une diffusion du principe actif étalé dans le temps. Ils délivrent une concentration du principe actif en plateau : la concentration est stable au cours du temps, on n'observe pas de pic (Cf. tests de pharmacodynamique).

L'invention a plus particulièrement pour objet trois types de matrice :
1 - une matrice monocouche,
2 - une matrice bicouche,
3 - une matrice tricouche qui sont décrites ci-après.

### 1 - Matrice monocouche

L'invention a plus particulièrement pour objet une matrice polymère adhésive appliquée à un dispositif destiné à l'administration transdermique d'un progestomimétique telle que définie précédemment caractérisée en ce que cette matrice renferme une seule couche (2) constituée d'un polymère silicone chargé en Trimegestone et/ou en un ou plusieurs dérivés pharmaceutiquement acceptables et éventuellement en plastifiant.

On choisit alors plus particulièrement le polymère silicone adhésif commercial ayant un pouvoir adhésif instantané fort. Il s'agira alors de BIO-PSA® ayant un pouvoir adhésif instantané fort et notamment le BIO-PSA® 7-4301.

Il n'est pas nécessaire d'utiliser un quelconque promoteur d'absorption.

L'invention a tout particulièrement pour objet la matrice monocouche précédente caractérisée en ce qu'elle est constituée de 80 à 99 % p/p de polymère silicone ayant un pouvoir adhésif fort chargé en 1 à 10 % p/p de Trimegestone et/ou en un ou plusieurs dérivés pharmaceutiquement acceptables et en 0 à 10 % p/p d'huile de silicone ou de Cetiol® S.

L'invention a tout particulièrement pour objet la matrice monocouche précédente caractérisée en ce qu'elle est constituée de 96 % p/p de polymère silicone BIO-PSA® ayant un pouvoir adhésif instantané fort chargé en 3 % p/p de Trimegestone et en 1 d p/p d'huile de silicone.

### 2 - Matrice bicouche

L'invention a également plus particulièrement pour objet une matrice polymère adhésive appliquée à un dispositif destiné à l'administration transdermique d'un progestomimétique telle que définie précédemment caractérisée en ce que cette matrice renferme deux couches successives :
a) une première couche (2), constituée d'un polymère silicone chargé en Trimegestone et/ou en un ou plusieurs dérivés pharmaceutiquement acceptables,
b) une deuxième couche (3), couche d'adhésion, qui sera en contact avec la peau, également constituée d'un polymère silicone.

En ce qui concerne la couche chargée et la couche d'adhésion, on choisit plus particulièrement le polymère silicone adhésif commercial ayant un pouvoir adhésif instantané fort. Il s'agira alors de BIO-PSA® ayant un pouvoir adhésif instantané fort et notamment le BIO-PSA® 7-4301.

L'ajout d'une couche d'adhésion, qui doit se trouver sur la peau, dans la formule bicouche, a permis d'augmenter l'adhésion, sans toutefois modifier de manière significative le flux. Il n'est ainsi pas nécessaire d'utiliser un quelconque promoteur d'absorption.

L'invention a également tout particulièrement pour objet la matrice bicouche précédente caractérisée en ce que
a) la première couche, est constituée de 90 à 99 % p/p d'un polymère silicone ayant un pouvoir adhésif fort chargé en 1 à 10 % p/p de Trimegestone et/ou en un ou plusieurs dérivés pharmaceutiquement acceptables,
b) la deuxième couche, est également constituée d'un polymère silicone ayant un pouvoir adhésif fort.

L'invention a également tout particulièrement pour objet la matrice bicouche précédente caractérisée en ce que
a) la première couche, est constituée de 97 % p/p d'un polymère silicone BIO-PSA® ayant un pouvoir adhésif instantané fort, chargé en 3 % p/p de Trimegestone,
b) la deuxième couche, est également constituée d'un polymère silicone BIO-PSA ayant un pouvoir adhésif instantané fort.

### 3 - Matrice tricouche

L'invention a aussi plus particulièrement pour objet une matrice polymère adhésive appliquée à un dispositif destiné à l'administration transdermique d'un progestomimétique telle que définie précédemment caractérisée en ce que cette matrice renferme trois couches successives :
a) une première couche (1), dite couche d'ancrage, constituée d'un polymère silicone,
b) une deuxième couche (2) constituée d'un polymère silicone chargé en Trimegestone et/ou en un ou plusieurs dérivés pharmaceutiquement acceptables,
c) et une troisième couche (3), couche d'adhésion, qui doit se trouver sur la peau, constituée elle aussi d'un polymère silicone.

L'ajout de la couche d'ancrage et de la couche d'adhésion a permis d'augmenter l'adhésion, sans toutefois modifier de manière significative le flux. Il n'est ainsi pas nécessaire d'utiliser un quelconque promoteur d'absorption.

En ce qui concerne la couche d'adhésion, on choisit plus particulièrement le polymère silicone adhésif commercial ayant un pouvoir adhésif instantané fort. Il s'agira alors de BIO-PSA® ayant un pouvoir adhésif instantané fort et notamment le BIO-PSA® 7-4301. En ce qui concerne la couche d'ancrage ainsi que la couche chargée on choisit plus particulièrement le polymère silicone à pouvoir adhésif instantané moyen et notamment le BIO-PSA® 7-3045, 7-4201 ou 7-4202.

L'invention a aussi tout particulièrement pour objet la matrice tricouche précédente caractérisée en ce que
- la première couche, est constituée d'un polymère silicone ayant un pouvoir adhésif moyen,
- la deuxième couche, est constituée de 90 à 99 % p/p de polymère silicone ayant un pouvoir adhésif moyen chargé en 1 à 10 % p/p de Trimegestone et/ou en un ou plusieurs dérivés pharmaceutiquement acceptables,
- la troisième couche, est constituée d'un polymère silicone ayant un pouvoir adhésif fort.

L'invention a aussi tout particulièrement pour objet la matrice tricouche précédente caractérisée en ce que
- la première couche est constituée d'un polymère silicone BIO-PSA® ayant un pouvoir adhésif instantané moyen,
- la deuxième couche est constituée de 91 % p/p de polymère silicone BIO-PSA® ayant un pouvoir adhésif instantané moyen chargé en 9 % p/p de Trimegestone,
- la troisième couche est constituée d'un polymère silicone BIO-PSA® ayant un pouvoir adhésif instantané fort.

L'invention a également pour objet un dispositif destiné à l'administration transdermique d'un progestomimétique caractérisé en ce qu'il est constitué successivement :
- d'un film de protection (a),
- d'une matrice, chargée en Trimegestone et/ou en un ou plusieurs dérivés pharmaceutiquement acceptables, telle que définie précédemment,
- d'un film protecteur pelable (b).

Les films de protections utilisés sont des supports sur lesquels sont enduites les différentes couches constituant le patch pour obtenir un système indissociable. Ils peuvent éventuellement être opaque, dans le cas où le principe actif est sensible à la lumière.

Parmi les films de protection, on choisit, de préférence le Scotchpak® 1109 qui est un film qui a la couleur de la peau, occlusif et souple, ou le Scotchpak® 1006 (Société 3M Health Care Limited).
Le Scotchpak® 1006 est constitué de 4 couches :
a) une couche pigmentée couleur chair
b) une couche d'aluminium
c) une couche de polyéthylène téréphtalate
d) une couche de polyéthylène densité moyenne et d'acétate d'éthylène.

Parmi les autre films de protections connus de l'homme du métier et applicables à la présente invention, on peut également citer le Hostaphan® de la gamme RN (RN23, RN25, RN75 ou RE75).

Les films protecteurs pelables utilisés sont des films destinés à préserver la face adhésive à placer sur la peau du système transdermique après fabrication et durant le stockage.

Parmi les films protecteurs pelables connus de l'homme du métier, on utilisera de préférence un film polyester dont une des faces est traitée par des fluorocarbones tel que le Scotchpak® 1022 (société 3M Health Care Limited) ou un film polyester transparent Silox® B5Y/O (société Akrosil™) dont une des faces est traitée antiadhérente par des silicones Bio-release de Dow-Corning.

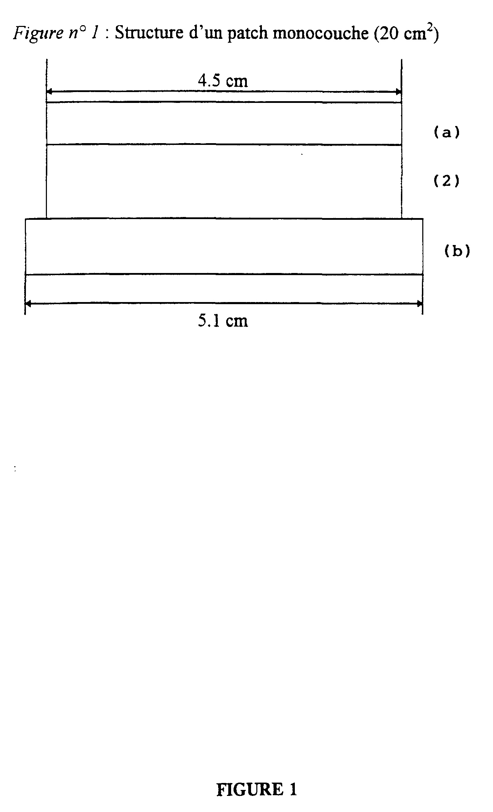

L'invention a plus particulièrement pour objet les dispositifs tels que décrits précédemment renfermant successivement (figure 1) :
- un film de protection (a),
- une matrice monocouche (2) telle que décrite précédemment,
- un film protecteur pelable (b).

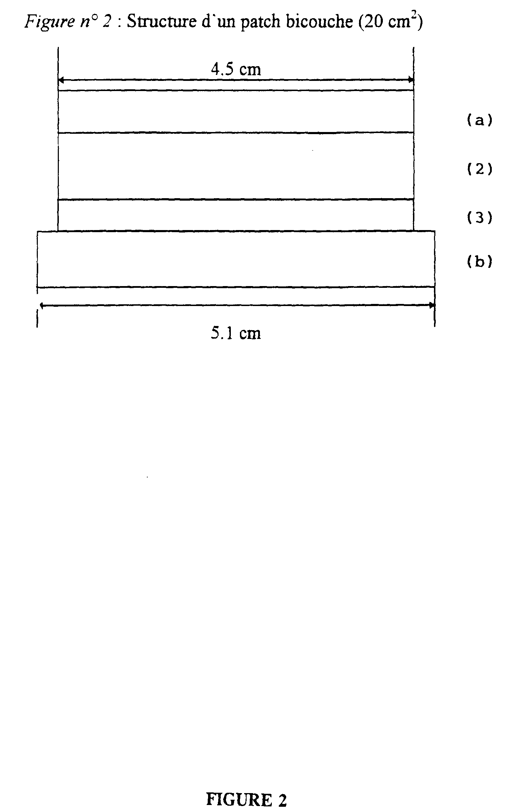

L'invention a plus particulièrement pour objet les dispositifs tels que décrits précédemment renfermant successivement (figure 2) :
- un film de protection (a),
- une matrice bicouche ((2) et (3)) telle que décrite précédemment,
- un film protecteur pelable (b).

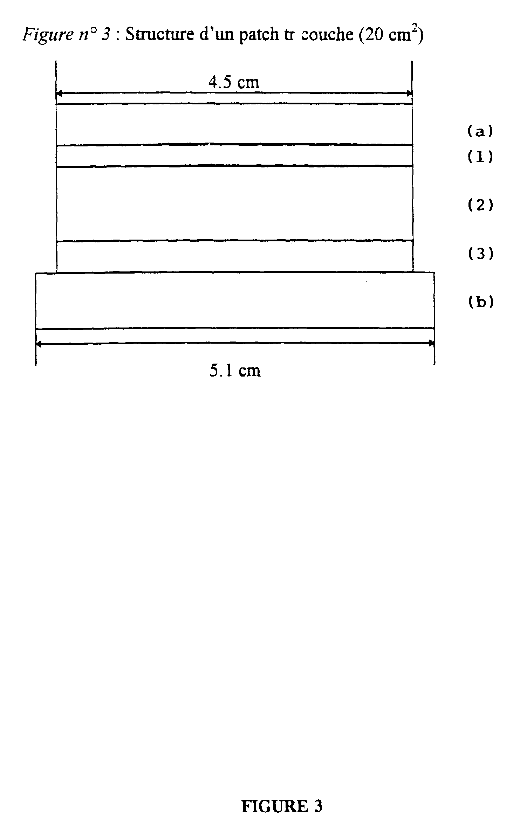

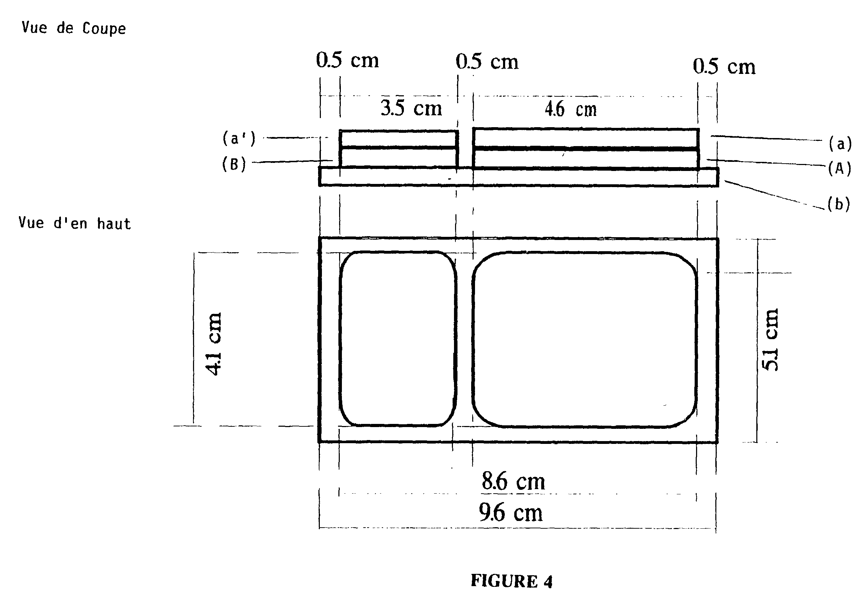

L'invention a plus particulièrement pour objet les dispositifs tels que décrits précédemment renfermant successivement (figure 3) :
- un film de protection (a),
- une matrice tricouche ((1), (2) et (3)) telle que décrite précédemment,
- un film protecteur pelable (b).
   A titre préférentiel les films de protection sont des films de protection opaque Scotchpak® 1006 et les films protecteurs pelables sont des films protecteurs pelables Scotchpak 1022.
   Ces systèmes transdermiques permettent de délivrer la Trimegestone avec un flux transcutané ex vivo sur peau humaine compris entre 0,1 et 3 µg·cm⁻²·h⁻¹.
   Les dispositifs tels que décrits précédemment peuvent être de forme quelconque : ronde, ovale, rectangulaire ou carrée. Leur surface est comprise entre 5 et 50 cm².
   La Trimegestone peut être associée avec un estrogène.
   L'invention a ainsi également pour objet un dispositif destiné à l'administration transdermique d'un progestomimétique tel que défini précédemment, caractérisé en ce que, en outre, il renferme une matrice chargée en estrogène, ce dispositif étant constitué de deux compartiments (A) et (B).
   Parmi les estrogènes préférés on peut citer le 17-béta-estradiol, les esters du 17-béta-estradiol tels que l'estradiol valérate, cyprionate, décanoate et acétate, l'éthynyl estradiol, l'oestrone, un estrogène "d'origine équine" tel que le Premarin® , ou une combinaison de ces composés.
   L'invention a plus spécialement pour objet le dispositif tel que décrit précédemment caractérisé en ce que le composé estrogène est le 17β-estradiol.
   L'invention a plus spécialement pour objet le dispositif tel que décrit précédemment caractérisé en ce que les deux compartiments (A) et (B)
- sont supportés par le même film protecteur pelable (b),
- et sont séparés l'un de l'autre par un espace vide ou une barrière de 1 à 10 mm ,
- le compartiment (A) renfermant la matrice polymère silicone, chargée en trimegestone et/ou en un ou plusieurs dérivés pharmaceutiquement acceptables, telle que décrite précédemment,
- le compartiment (B) renfermant une matrice polymère adhésive chargée en estrogène,
- et chacune de ces matrices étant respectivement recouverte d'un film de protection (a) et (a') identique ou différent (figure 4).

Le compartiment (A) a une surface comprise entre 5 et 50 cm² et le compartiment (B) a une surface comprise entre 5 cm² et 50 cm².

Le film protecteur pelable supporte ainsi deux compartiments indépendants (A) et (B) renfermant respectivement la Trimegestone et un composé estrogène.

Une fois ce film protecteur pelable retiré, on obtient deux patchs indépendants qu'il s'agira d'appliquer sur la peau ou une muqueuse de façon à avoir une administration simultanée et séparée de la Trimegestone et du composé estrogène. On peut également prévoir un ou plusieurs moyens de fixation entre les deux compartiments (A) et (B) afin que les deux patchs demeurent solidaires une fois le film protecteur pelable retiré.

La matrice polymère renfermant le composé estrogène est choisie parmi des polymères du commerce et/ou connus de l'homme du métier. Il s'agit notamment des polymères ou copolymères constitués par un réseau de chaînes polyisobutylène ou polyacrylique, les copolymères d'éthylène et d'acétate de vinyle (EVA) ou encore les polymères silicone. Le cas échéant on peut ajouter à ces polymères, un polymère hydrophile et/ou un promoteur d'absorption et/ou un plastifiant et/ou d'autres additifs connus de l'homme du métier permettant d'améliorer les critères de flux, d'adhésion et de stabilité des systèmes transdermiques.

Cette matrice peut être mono ou multicouches. Il peut également s'agir d'un système à réservoir.

L'espace vide permettant de séparer les deux compartiments peut être compris entre 1 et 10 mm. Il est de préférence compris entre 2 et 4 mm.

On entend par barrière, un séparateur physique constitué d'un matériau adapté. Sa largeur est définie par rapport à son efficacité à stopper la diffusion et par le procédé de fabrication. Sa largeur peut être comprise entre 1 et 10 mm. Elle sera de préférence comprise entre 1 et 3 mm.

Lorsque le composé estrogène est l'estradiol, la matrice chargée est préférentiellement une matrice monocouche constituée d'un mélange d'acrylate de 2-éthylhexyle et d'acétate de vinyle, chargé en estradiol à laquelle on adjoint éventuellement un polymère hydrophile. Il s'agira alors tout particulièrement du copolymère Gelva® 737 renfermant 72 % de 2-éthylhexylacrylate et 28 % de vinylacétate. Le polymère hydrophile sera de préférence la polyvinylpyrrolidone. Il s'agira tout particulièrement du Kollidon® 30 ou 90F.

La quantité d'estradiol incorporée dans une matrice polymère telle que définie précédemment est de préférence comprise entre 1 % p/p et 10 % p/p.

L'invention a tout particulièrement pour objet les dispositifs tels que décrits précédemment présentant les caractéristiques suivantes :
**BIPATCH 1** constitué
   - d'un film protecteur pelable (b) supportant les deux compartiments (A) et (B), séparés l'un de l'autre par un espace vide ou une barrière de 1 à 10 mm,
   - le compartiment (A) renfermant une matrice monocouche, recouverte d'un film de protection (a), et constituée de polymère silicone chargé en Trimegestone et/ou en un ou plusieurs dérivés pharmaceutiquement acceptables, et éventuellement en plastifiant,
   - et le compartiment (B) renfermant une matrice monocouche, recouverte d'un film de protection (a'), et constituée d'un copolymère d'acrylate de 2-éthylhexyle et d'acétate de vinyle, chargé en estradiol, et éventuellement en polymère hydrophile.
**BIPATCH 2** constitué
   - d'un film protecteur pelable (b) supportant les deux compartiments (A) et (B), séparés l'un de l'autre par un espace vide ou une barrière de 1 à 10 mm,
   - le compartiment (A) renfermant une matrice bicouche recouverte d'un film de protection (a),
      a) la première couche, étant constituée d'un polymère silicone chargé en Trimegestone et/ou en un ou plusieurs dérivés pharmaceutiquement acceptables,
      b) la deuxième couche, couche d'adhésion à la peau, étant également constituée d'un polymère silicone,
   - et le compartiment (B) renfermant une matrice monocouche, recouverte d'un film de protection (a'), et constituée d'un copolymère d'acrylate de 2-éthylhexyle et d'acétate de vinyle, chargé en estradiol et éventuellement en polymère hydrophile.
      L'invention a tout particulièrement pour objet les dispositifs tels que décrits précédemment renfermant :
**BIPATCH 1a** constitué
   - d'un film protecteur pelable (b) supportant les deux compartiments (A) et (B), séparés l'un de l'autre par un espace vide ou une barrière de 1 à 10 mm,
   - le compartiment (A) renfermant une matrice monocouche recouverte d'un film de protection opaque (a) et constituée de 80 à 99 % p/p de polymère silicone ayant un pouvoir adhésif fort chargé en 1 à 10 % p/p de Trimegestone et/ou en un ou plusieurs dérivés pharmaceutiquement acceptables et en 0 à 10 % p/p d'huile de silicone ou de Cetiol® S,
   - et le compartiment (B), renfermant une matrice monocouche recouverte d'un film de protection (a') et constituée de 60 à 99 % p/p de Gelva® 737 chargé en 1 à 10 % p/p d'estradiol et en 0 à 30 % p/p de Kollidon® .
**BIPATCH 2a** constitué
   - d'un film protecteur pelable (b) supportant les deux compartiments (A) et (B), séparés l'un de l'autre par un espace vide ou une barrière de 1 à 10 mm,
   - le compartiment (A) renfermant une matrice bicouche recouverte d'un film de protection (a),
      a) la première couche, étant constituée de 90 à 99 % p/p d'un polymère silicone ayant un pouvoir adhésif fort, chargé en 1 à 10 % p/p de Trimegestone et/ou en un ou plusieurs dérivés pharmaceutiquement acceptables,
      b) la deuxième couche, couche d'adhésion à la peau, étant également constituée d'un polymère silicone ayant un pouvoir adhésif fort,
   - et le compartiment (B) renfermant une matrice monocouche, recouverte d'un film de protection (a') et constituée de 60 à 99 % p/p de Gelva® 737 chargé en 1 à 10 % p/p d'estradiol et en 0 à 30 % p/p de Kollidon® 90F.
Ce "bipatch" permet :
   - de réunir dans une même entité la Trimegestone et un composé estrogène devant être administrés de manière simultanée, séparée et étalée dans le temps pour le traitement hormonal substitutif de la ménopause et en particulier dans la prévention ou le traitement de l'ostéoporose,
   - de régler les problèmes de différence de stabilité des principes actifs au sein des polymères employés pour les couches chargées : la Trimegestone n'est pas stable dans la matrice employée pour l'estradiol,
   - d'administrer chaque principe actif dans les conditions optimum pour obtenir un flux transcutané pharmaceutiquement acceptable et évite toute interaction d'un composé avec la matrice de l'autre composé,
   - de répondre aux prescriptions requises en matière de doses et du jour d'administration de l'un et l'autre principe actif (prédosage), tout en évitant l'achat et la manipulation de deux patchs individuels.

Dans le cadre d'une association estro-progestative, ce dernier point est particulièrement important pour le traitement de hormonal substitutif de la ménopause et en particulier la prévention ou le traitement de l'ostéoporose ou pour un traitement contraceptif.

Il présente en outre les avantages suivants : en appliquant sur le même film protecteur pelable deux matrices indépendantes, on peut aisément :
- optimiser de manière indépendante les formulations renfermant les principes actifs (choix du polymère support, choix d'un promoteur d'absorption, choix du polymère hydrophile, choix d'un plastifiant, optimisation du grammage), en fonction des critères d'adhésion et de flux désirés,
- optimiser de manière indépendante les concentrations des principes actifs en fonction de critères de stabilité, de flux transcutanés désirés ainsi que des doses prescrites,
- obtenir des compartiments ayant des tailles identiques ou différentes, ceci par un procédé de fabrication simple.

L'invention a également pour objet un dispositif ("combipatch") constitué successivement des éléments suivants:
un film de protection (a),
un compartiment (B) constitué d'une matrice polymère adhésive chargée en estrogène tel que l'estradiol,
un film polyester (c) ayant les mêmes dimensions que le compartiment (A) et superposé à celui-ci,
un compartiment (A) constitué de la matrice polymère silicone chargée en Trimegestone et/ou en plusieurs dérivés pharmaceutiquement acceptables, telle que définie ci-dessus, le compartiment (A) étant de dimension inférieure au compartiment (B) telle que la moitié et étant de préférence centré par rapport à ce compartiment (B),
un film de protection pelable (b).

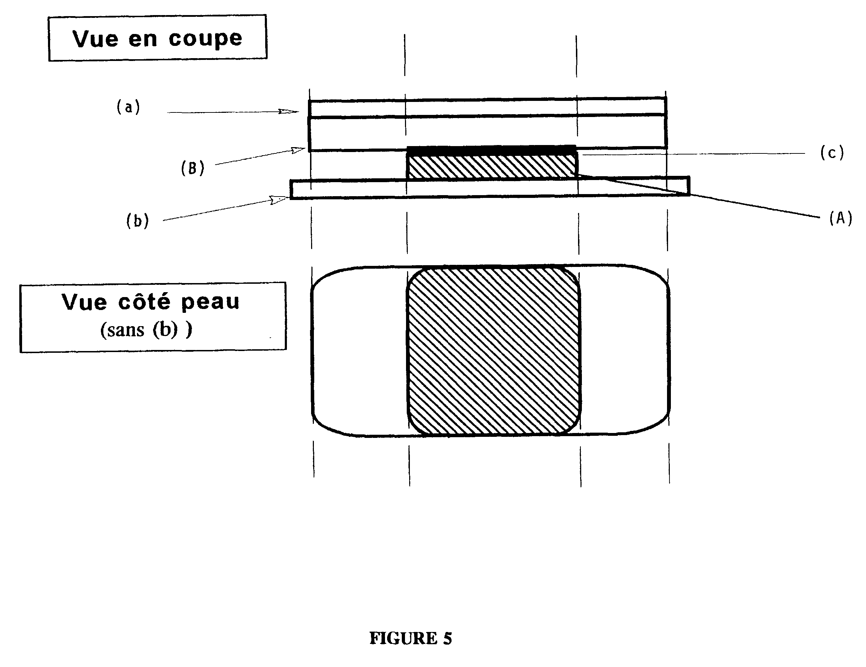

Le compartiment (A) est de dimension inférieure au compartiment (B) afin que la matrice chargée en estrogène, située au dessus de la matrice chargée en Trimegestone, puisse également être en contact direct avec la peau. Le composé estrogène diffusera ainsi directement par cet endroit. Par exemple, le compartiment (A) peut avoir une surface de 15 cm² et le compartiment (B) une surface de 30 cm² (voir Figure 5).

A titre préféré, le compartiment (A) est constitué d'une matrice bicouche ou tricouche telle que définie précédemment.

Le "combitatch" ayant une matrice bicouche peut être utilisé par exemple 4 jours et le "combipatch" ayant une matrice tricouche peut être utilisé par exemple 7 jours.

La technique de fabrication des systèmes transdermiques objet de l'invention est l'enduction. Le principe général est le suivant :

On mélange une solution de polymère silicone dans un solvant organique apolaire tel que l'heptane avec le principe actif et tout autre additif, et on obtient une suspension ou une solution d'adhésif (2) que l'on étale (enduction) sur un film protecteur pelable ou un film de protection. Après éventuellement une pré-évaporation sous hotte aspirante à température ambiante, le film est séché à une température comprise entre 40°C et 100°C jusqu'à évaporation complète du solvant. On réitère ce type d'opération dans un ordre approprié en fonction du nombre de couches désirées puis on découpe le système transdermique en pastilles, appelées patchs, les surfaces des ces patchs pouvant aller de 1 à 50 cm². On entend par solution d'adhésif (1) ou (3) des solutions de polymère silicone dans un solvant organique apolaire tel que l'heptane non chargées en principe actif.

Le patch "monocouche" peut être préparé de la façon suivante :
1 - on dépose préalablement sur un film protecteur pelable (b), une solution de couche adhésive (2) dans l'heptane chargée en Trimegestone et éventuellement en plastifiant, puis on sèche,
2 - on effectue une opération de colaminage de l'ensemble "couche adhésive (2)/film protecteur pelable (b)" sur un film de protection (a),
3 - on découpe les systèmes transdermiques en pastille ayant une surface comprise entre 5 cm² à 50 cm².

On obtient ainsi le patch caractérisé en ce qu'il renferme (figure 1) :
- un film de protection (a)
- une couche adhésive (2) chargée en Trimegestone et éventuellement en plastifiant,
- un film protecteur pelable (b).

Le procédé de fabrication peut également s'opérer ainsi
1 - on dépose préalablement sur un film de protection (a), une solution de couche adhésive dans l'heptane chargée en Trimegestone et éventuellement en plastifiant (2), puis on sèche,
2 - on effectue une opération de colaminage de l'ensemble "couche adhésive (2)/film de protection (a)" sur un film protecteur pelable (b),
3 - on découpe les systèmes transdermiques en pastille ayant une surface comprise entre 5 cm² à 50 cm².

Le patch "bicouche" peut être préparé de la façon suivante :
1 - on dépose préalablement
   a) sur un film protecteur pelable (b), une solution de couche adhésive (3) dans l'heptane, puis on sèche,
   b) sur un film protecteur pelable provisoire (b'), une solution de couche adhésive (2) dans l'heptane chargée en Trimegestone, puis on sèche,
2 - on effectue une opération de colaminage de l'ensemble "couche adhésive (2) chargée en Trimegestone/film protecteur pelable provisoire (b')" sur l'ensemble "couche adhésive (3)/film protecteur pelable (b)",
3 - on effectue un pelage du film protecteur provisoire (b'),
4 - on effectue une opération de colaminage de l'ensemble "couche adhésive (2)/couche adhésive (3)/film protecteur pelable (b)" sur un film de protection (a),
5 - on découpe les systèmes transdermiques en pastille ayant une surface comprise entre 5 cm² à 50 cm².

On obtient ainsi le patch caractérisé en ce qu'il renferme (figure 2) :
- un film de protection (a),
- une couche adhésive chargée en Trimegestone (2),
- une couche d'adhésion entre la couche chargée et le film protecteur pelable (3),
- un film protecteur pelable (b).

Le patch "tricouche" peut être préparé de la façon suivante :
1 -
   a) on dépose préalablement sur un film protecteur pelable provisoire (b'), une solution de couche adhésive (1) dans l'heptane, puis on sèche,
   b) on dépose préalablement sur un film protecteur pelable provisoire (b"), une solution de couche adhésive (2) dans l'heptane chargée en Trimegestone, puis on sèche,
   c) on dépose préalablement sur un film protecteur pelable (b), une solution de couche adhésive (3) dans l'heptane, puis on sèche,
2 - on effectue une opération de colaminage de l'ensemble "couche adhésive (1)/film protecteur pelable provisoire (b')" sur un film de protection (a),
3 - on effectue un pelage du film protecteur provisoire (b')
4 - on effectue une opération de colaminage de l'ensemble "couche adhésive (2) chargée en Trimegestone/film protecteur pelable provisoire (b")" sur l'ensemble "couche adhésive (1)/film de protection (a)",
5 - on effectue le pelage du film protecteur provisoire (b"),
6 - on effectue enfin une dernière opération de colaminage du système "couche adhésive (3)/film protecteur pelable (b)" sur l'ensemble "couche chargée (2)/couche adhésive (1)/film de protection (a)".
7 - on découpe les systèmes transdermiques en pastille ayant une surface comprise entre 5 cm² à 50 cm².

On obtient ainsi le patch caractérisé en ce qu'il renferme (figure 3) :
- un film protecteur pelable (b),
- une couche d'adhésion entre le film de protection et la couche chargée (3),
- une couche adhésive chargée en Trimegestone (2),
- une couche d'adhésion entre la couche chargée et le film de protection (couche d'ancrage) (1),
- un film de protection (a).

Le dispositif "bipatch" permettant l'administration transdermique de la Trimegestone associée à un estrogène tel que décrit précédemment peut être préparé de la manière suivante :
**Etape I :** pour la fabrication du patch correspondant au compartiment (A)
   1 - on enduit la couche polymère adhésive silicone chargée en Trimegestone et éventuellement en un ou plusieurs additifs, sur le film de protection (a),
   2 - on évapore le solvant jusqu'à obtention de l'ensemble "matrice chargée en Trimegestone/film protecteur (a)" correspondant au compartiment (A),
   3 - on effectue une opération de colaminage de l'ensemble "matrice chargée en Trimegestone/film protecteur (a)" sur un film protecteur pelable (b'),
   4 - on découpe un patch de 5 à 50 cm².
**Etape II :** pour la fabrication du patch correspondant au compartiment (B)
   1 - on enduit la couche polymère adhésive chargée en composé estrogène et éventuellement en un ou plusieurs additifs tels qu'un polymère hydrophile, un promoteur d'absorption ou un plastifiant, sur le film de protection (a'),
   2 - on évapore le solvant jusqu'à obtention de l'ensemble "matrice chargée en estrogène/film protecteur (a')" correspondant au compartiment (B),
   3 - on effectue une opération de colaminage de l'ensemble "matrice chargée en estrogène/film protecteur (a')" sur film protecteur pelable (b'),
   4 - on découpe un patch de 5 à 50 cm².
**Etape III :** pour la formation du "bipatch"
   1 - on effectue un pelage du film protecteur pelable (b') du patch obtenu à l'étape I,
   2 - puis on transfert l'ensemble "matrice chargée en Trimegestone/film protecteur (a)" sur un film protecteur pelable (b),
   3 - on effectue un pelage du film protecteur pelable (b") du patch obtenu à l'étape II,
   4 - puis on transfert l'ensemble matrice chargée en "estrogène/film protecteur (a')" sur le film protecteur pelable (b) précédent, en respectant une distance de 1 à 10 mm ou après avoir introduit une barrière entre les deux compartiments (A) et (B).

On obtient ainsi le "bipatch" caractérisé en ce qu'il renferme (figure 4) :
- un film protecteur pelable (b),
- un compartiment (A) constitué d'une matrice chargée en trimegestone telle que définie précédemment et recouverte d'un film protecteur (a)
- un compartiment (B) constitué d'une matrice chargée en un composé estrogène et recouverte d'un film protecteur (a'), les deux compartiments étant séparés l'un de l'autre par un espace vide ou une barrière de 1 à 10 mm.

L'ensemble de ces procédés peut s'appliquer également aux esters de la Trimegestone tels que définis précédemment.

Ces différents procédés sont dans tous les cas moins coûteux que les procédés de fabrication des systèmes à réservoir. Ce sont des procédés simples à mettre en oeuvre et qui nécessitent des temps de séchage assez courts. De préférence, les séchages s'effectuent à 60°C pendant 15 mn.

Le séchage est réalisé en continu en procédé industriel. Les conditions de fabrication sont les suivantes :
segment I : 35°C, segment II : 50°C, segment III : 60°C et
segment IV : 80°C.

Le procédé de fabrication du patch monocouche est particulièrement simple car d'une part il n'y a qu'une seule couche, ensuite il n'y pas de perte en film de transfert nécessaires lors des opérations de colaminage et enfin une simplification des réglages par rapport à un colaminage. Il est peu coûteux pour les raisons citées plus haut, mais encore car il permet une grande rapidité de fabrication et il nécessite moins de matière première (Trimegestone et excipients). Le procédé de fabrication du patch bicouche présente l'avantage par rapport au monocouche de ne pas nécessiter l'apport d'un agent plastifiant.

La Trimegestone possède une très bonne affinité vis-à-vis du récepteur de la progestérone (6 à 7 fois celle de la progestérone). Il présente par ailleurs une très faible affinité vis-à-vis du récepteur androgène et aucune affinité vis-à-vis du récepteur estrogène (<0,02). Les études in vivo ont confirmé les études in vitro. La Trimegestone est un puissant progestomimétique dénué d'activité minéralocorticoïde, androgène, glucocorticoïde, antiglucocorticoïde et estrogène. Par contre elle présente une activité antiminéralocorticoïde et antiandrogène. La Trimegestone sous forme patch présente ainsi un grand intérêt en thérapeutique.

Le patch renfermant la Trimegestone selon l'invention peut être utilisé dans le traitement des troubles gynécologiques dus à une insuffisance lutéale :
- troubles menstruels et/ou du cycle,
- dysménorrhées,
- syndrome prémenstruel,
- mastodynie, mastopathie,
- hémorragies fonctionnelles
- ménorragies des fibromes,
- hyperplasie endométriale,
- troubles de la préménopause,
- endométriose,
- troubles de la ménopause,
- contraception,
- dystrophies ovariennes par mise au repos des ovaires,
- le traitement des tumeurs et de l'utérus.

En association avec un estrogène tel que l'estradiol, le progestatif Trimegestone manifeste une forte activité antiestrogène au niveau de l'utérus tout en ne manifestant aucune activité antiestrogène au niveau osseux. L'association estrogène/Trimegestone selon l'invention trouve donc une application dans le traitement hormonal substitutif de la ménopause et en particulier dans la prévention ou le traitement de l'ostéoporose.

Parmi les estrogènes préférés on peut citer le 17-béta-estradiol, les esters du 17-béta-estradiol tels que l'estradiol valérate, cyprionate, décanoate et acétate, l'éthynyl estradiol, l'oestrone, l'estrogène "d'origine équine" tel que le Premarin® , ou une combinaison de ces composés.

L'ostéoporose, quand à elle, est une pathologie qui se caractérise par une réduction quantitative et qualitative du tissu osseux, suffisante pour entraîner des fractures vertébrales ou périphériques, ce de façon spontanée ou à l'occasion de traumatismes minimes. Bien que cette affection soit d'origine multifactorielle, c'est la ménopause qui, chez la femme, constitue le facteur prépondérant de la perte osseuse ou ostéopénie.

Cette ostéopénie se manifeste par une raréfaction et une modification de l'architecture de l'os spongieux qui a pour conséquence d'accentuer la fragilité squelettique et le risque fracturaire. La perte osseuse s'accentue fortement après la ménopause en raison de la suppression de la fonction ovarienne et atteint 3 à 5 % par an pour se ralentir après 65 ans.

Dans un but thérapeutique, la carence hormonale post-ménopausique peut être compensée par une hormonothérapie substitutive où l'estrogène joue un rôle majeur en préservant le capital osseux. Mais l'estrogénothérapie au long cours s'accompagne parfois d'effets indésirables sur l'appareil génital (hyperplasie endométriale, tumeurs mammaires ...), ce qui constitue un inconvénient majeur et limite son application. Il convient dès lors d'associer à l'estrogène, un progestatif qui soit capable de s'opposer aux effets secondaires de l'estrogène sur les cibles génitales tout en maintenant son action bénéfique sur le tissu osseux. Des associations de ce type sont déjà connues et sont décrites par exemple dans les brevets ou les demandes de brevet suivants EP-0136011, US5208225, US5108995, EP-474374, DE4019670. Elles présentent toutefois un important inconvénient dû à la multiplicité des activités des progestatifs utilisés dans les associations et notamment à leurs effets androgènes.

L'association ne présente pas cet inconvénient. En effet la trimegestone qui appartient à la classe des progestatifs norpregnane, est pratiquement dénuée de toute activité androgène, ce qui entraîne une bonne tolérance métabolique.

L'association estrogène/Trimegestone selon l'invention trouve également son application à titre de contraceptif. L'estrogène sera alors tout particulièrement l'éthynylestradiol.

L'invention a donc pour objet le dispositif tel que décrit précédemment pour son utilisation, dans un procédé de délivrance, soit de la Trimegestone et/ou un ou plusieurs dérivés pharmaceutiquement acceptable soit de la Trimegestone associée à un estrogène, à un patient par application de la ou des matrices du dispositif à la peau ou à une muqueuse dudit patient.

Les exemples de traitement ci-dessous illustrent l'invention sans toutefois la limiter.

### 1) Trimegestone seule :

### Traitement a

Un premier patch peut être appliqué du 16ème au 18ème jour du cycle, un second patch du 19ème au 21ème jour du cycle et un troisième patch du 22ème au 25ème jour du cycle, soit 10 jours par cycle.

### Traitement b

Un premier patch peut être appliqué du 5ème au 8ème jour du cycle, un second patch du 9ème au 12ème jour du cycle, un troisième patch du 13ème au 16ème jour du cycle, un quatrième patch du 17ème au 21ème jour du cycle, un cinquième patch du 22ème au 25ème jour du cycle, soit 21 jours par cycle.

Un traitement en continu de la Trimegestone peut aussi être utilisé.

### 2) Trimegestone associée avec l'estradiol

Dans le cadre d'un traitement hormonal substitutif de la ménopause et en particulier dans la prévention ou le traitement de l'ostéoporose. L'estradiol peut se trouver sous la forme comprimé ou sous la forme patch.

### Administration de la Trimegestone en séquentiel et de l'estradiol en continu :

### Traitement a

Administration de l'estradiol en continu (cycles de 28 jours sans interruption entre les cycles) à une dose de 25 à 200 µg par jour et de la Trimegestone les 14 derniers jours de chaque cycle de 28 jours, à une dose de 0,05 à 2,5 mg par jour.

### Traitement b

Administration de l'estradiol 28 jours par mois à une dose de 25 à 200 µg par jour et de la Trimegestone les 14 derniers jours de l'administration de l'estradiol à une dose de 0,05 à 2,5 mg par jour. Le traitement est arrêté 2 à 3 jours par mois à la fin de chaque cycle de 28 jours.

### Traitement c

Administration de l'estradiol 28 jours par mois à une dose de 25 à 200 µg par jour et de la Trimegestone les 14 premiers jours de l'administration de l'estradiol à une dose de 0,05 à 2,5 mg par jour. Le traitement est administré soit sans interruption entre chaque cycle de 28 jours, soit avec interruption de 2 à 3 jours par mois à la fin de chaque cycle.

### Traitement d

Administration de l'estradiol 25 jours par mois à une dose de 25 à 200 µg par jour et de la Trimegestone à une dose de 0,05 à 2,5 mg par jour pendant les 11 à 14 derniers jours de l'administration de l'estradiol. Le traitement est arrêté 5 à 6 jours à la fin de chaque cycle de 25 jours.

### Administration en continu de la Trimegestone et de l'estradiol

Administration continue de l'estradiol à une dose de 25 à 200 µg par jour et du patch trimegestone à une dose de 0,05 à 2,5 mg par jour. Il n'y a pas d'interruption de traitement.

### 3) Trimegestone associée à l'éthynylestradiol

### Dans le cadre d'un usage à titre de contraceptif.

On administre l'association en continu du patch Trimegestone et de l'éthynylestradiol de 21 à 28 jours par cycles. Ce traitement nécessite ainsi l'application successive de 3 à 8 patchs de Trimegestone et la prise de l'éthynyl estradiol de 21 à 28 jours/cycle notamment sous forme de patch ou de comprimés.

Des exemples de patchs selon l'invention figurent ci-après dans la partie expérimentale. Les exemples suivants illustrent l'invention sans toutefois la limiter.

### EXEMPLE 1 :

### Patch monocouche renfermant la Trimegestone

- Un film de protection opaque Scotchpak 1006 de 70 ± 2 µm d'épaisseur **(a),**
- Une couche de 94 % p/p de polymère silicone BIO-PSA chargé en 3 % p/p de Trimegestone et en 1 % p/p d'huile de silicone, de 50 à 60 µm d'épaisseur **(2),**
- Un film protecteur pelable Scotchpak 1022 de 70 ± 1 µm d'épaisseur **(b).**

Ce patch a une surface de 20 cm² et délivre ex vivo de 0,80 ± 0,54 µg.h⁻¹.cm⁻² de Trimegestone (voir figure 1). On a préparé les matrices suivantes :

### EXEMPLE 1a : (matrice monocouche)

Surface du patch 20 cm², Grammage 60 g/m²

| Composants | % p/p | mg/patch |
|---|---|---|
| Trimegestone | 3 | 3,6 |
| BIO-PSA® 7-4301 | 96 | 115,2 |
| Huile de silicone 7-9120 | 1 | 1,2 |
| Total | 100 | 120 |

### EXEMPLE 1b : (matrice monocouche)

Surface du patch 20 cm², Grammage 60 g/m²

| Composants | % p/p | mg/patch |
|---|---|---|
| Trimegestone | 3 | 3,6 |
| BIO-PSA® 7-4301 | 94 | 112,8 |
| Huile de silicone 7-9120 | 3 | 3,6 |
| Total | 100 | 120 |

### EXEMPLE 1c : (matrice monocouche)

Surface du patch 20 cm², Grammage 60 g/m²

| Composants | % p/p | mg/patch |
|---|---|---|
| Trimegestone | 3 | 3,6 |
| BIO-PSA® 7-4301 | 92 | 110,4 |
| Huile de silicone 7-9120 | 5 | 6,0 |
| Total | 100 | 120 |

### EXEMPLE 1d :

Surface du patch 20 cm², Grammage 40 g/m²

| Composants | % p/p | mg/patch |
|---|---|---|
| Trimegestone | 3 | 2,4 |
| BIO-PSA® 7-4301 | 94 | 75,2 |
| Huile de silicone 7-9120 | 3 | 2,4 |
| Total | 100 | 80 |

### EXEMPLE 1e :

Surface du patch 20 cm², Grammage 80 g/m²

| Composants | % p/p | mg/patch |
|---|---|---|
| Trimegestone | 3 | 4,8 |
| BIO-PSA® 7-4301 | 94 | 150,4 |
| Huile de silicone 7-9120 | 3 | 4,8 |
| Total | 100 | 160 |

### EXEMPLE 1f : (matrice monocouche)

Surface du patch 20 cm², Grammage 40 g/m²

| Composants | % p/p | mg/patch |
|---|---|---|
| Trimegestone | 3 | 2,4 |
| BIO-PSA® 7-4301 | 94 | 75,2 |
| Cétiol® S | 3 | 2,4 |
| Total | 100 | 80 |

### EXEMPLE 1g : (matrice monocouche)

Surface du patch 20 cm², Grammage 60 g/m²

| Composants | % p/p | mg/patch |
|---|---|---|
| Trimegestone | 3 | 3,6 |
| BIO-PSA® 7-4301 | 94 | 112,8 |
| Cétiol® S | 3 | 3,6 |
| Total | 100 | 120 |

### EXEMPLE 1h : (matrice monocouche)

Surface du patch 20 cm², Grammage 40 g/m²

| Composants | % p/p | mg/patch |
|---|---|---|
| Trimegestone | 3 | 4,8 |
| BIO-PSA® 7-4301 | 94 | 150,4 |
| Cétiol® S | 3 | 4,8 |
| Total | 100 | 160 |

### EXEMPLE 2 :

### Patch bicouche renfermant 1a Trimegestone

- Un film de protection opaque Scotchpak 1006 de 70 ± 2 µm d'épaisseur **(a)**
- Une couche de 97 % p/p de polymère silicone BIO-PSA® ayant un pouvoir adhésif instantané fort chargé en 3 % p/p de Trimegestone, de 50 à 60 µm d'épaisseur **(2),**
- Une couche d'adhésion BIO-PSA® ayant un pouvoir adhésif instantané fort de 65 µm d'épaisseur **(3),**
- Un film protecteur pelable Scotchpak 1022 de 70 ± 1 µm d'épaisseur **(b).**

Ce patch a une surface de 20 cm² et délivre ex vivo 1,29 ± 0,45 µg·h⁻¹·cm⁻² de Trimegestone (voir figure 2). On a préparé la matrice suivante :

### EXEMPLE 2a : (Matrice bicouche)

Surface du patch 20 cm², Grammage 60 + 30 g/m²

| Couche | Composants | % p/p | mg/patch |
|---|---|---|---|
| Matrice chargée | Trimegestone | 3 | 3,6 |
| | BIO-PSA® 7-4301 | 97 | 116,4 |
| Couche d'adhésion à la peau | BIO-PSA® 7-4301 | 100 | 60 |
| | Total | - | 180 |

### EXEMPLE 3 :

On a préparé le patch "tricouche" suivant :

Ce patch est constitué successivement des couches suivantes :
- Un film de protection opaque Scotchpak 1006 de 70 ± 2 µm d'épaisseur **(a),**
- Une couche d'ancrage BIO-PSA® ayant un pouvoir adhésif instantané moyen d'environ 33 µm d'épaisseur **(1),**
- Une couche de polymère silicone BIO-PSA® ayant un pouvoir adhésif instantané moyen (91 % p/p) chargé en Trimegestone (9 % p/p) de 50 à 60 µm d'épaisseur **(2),**
- Une couche d'adhésion BIO-PSA® ayant un pouvoir adhésif instantané fort d'environ 65 µm d'épaisseur **(3),**
- Un film protecteur pelable Scotchpak 1022 de 70 ± 1 µm d'épaisseur **(b).**

Ce patch a une surface de 20 cm² et délivre ex vivo 0,59 ± 0,33 µg·h⁻¹·cm⁻² de Trimegestone (voir figure 3). On a préparé la matrice suivante :

### EXEMPLE 3a : (Matrice tricouche)

Surface du patch 20 cm², Grammage 30,5 + 61,5 + 65 g/m²

| Couche | Composants | % p/p | mg/patch |
|---|---|---|---|
| Couche d'ancrage | BIO-PSA® 7-4201 | 100 | 61 |
| Matrice chargée | Trimegestone | 9 | 11,07 |
| | BIO-PSA® 7-4201 | 91 | 111,93 |
| Couche d'adhésion à la peau | BIO-PSA® 7-4301 | 100 | 130 |
| | Total | - | 314 |

### Exemple 4 :

### Patch renfermant la Trimegestone associée à l'estradiol

### BIPATCH 1b (cf figure 4)

Ce bipatch comporte les caractéristiques suivantes :
- un film protecteur pelable **(b)** Scotchpak® 1022 d'environ 70 µm d'épaisseur, caractérisé en ce qu'il supporte les deux compartiments (A) et (B), séparés l'un de l'autre par un espace vide de 2 à 4 mm
- le compartiment (A) renfermant une matrice monocouche d'environ 60 µm d'épaisseur, recouverte d'un film de protection opaque **(a)** Scotchpak® 1006 d'environ 70 µm d'épaisseur et constituée de 96 % p/p de BIO PSA® ayant un pouvoir adhésif instantané fort chargée en 3 % p/p de Trimegestone et en 1 % p/p d'huile de silicone (7-9120, 12000 cSt). Le grammage est égal à 60 g/m²,
- et le compartiment (B), renfermant une matrice monocouche, d'environ 76 µm d'épaisseur recouverte d'un film de protection **(a')** Scotchpack® 1109 d'environ 34 µm d'épaisseur ou Hostaphan® RN23 et constituée de 73 % p/p de Gelva® 737 chargé en 2 % p/p d'estradiol et en 25 % p/p de Kollidon® 90F. Le grammage est égal à 80 g/m².

### BIPATCH 2b

Ce bipatch comporte les caractéristiques suivantes :
- un film protecteur pelable **(b)** Scotchpak 1022 caractérisé en ce qu'il supporte les deux compartiments (A) et (B), séparés l'un de l'autre par un espace vide de 2 à 4 mm
- le compartiment (A) renfermant une matrice bicouche recouverte d'un film de protection **(a)** Scotchpak® 1006,
   a) la première couche, chargée en 3 % p/p de Trimegestone, étant constituée de 97 % p/p d'un polymère silicone BIO PSA® ayant un pouvoir adhésif instantané fort,
   b) la deuxième couche, couche d'adhésion à la peau, étant constituée d'un polymère silicone BIO PSA® ayant un pouvoir adhésif instantané fort.

Le grammage total est alors égal à 90 g/m²,
- et le compartiment (B) renfermant une matrice monocouche, recouverte d'un film de protection **(a')** Scotchpak® 1109 ou Hostaphan® RN23 constituée de 73 % p/p d'une couche Gelva® 737 chargé en 2 % p/p d'estradiol et en 25 % p/p de Kollidon® 90F. Le grammage est égal à 80 g/m².

### Tests sur la structure du patch

Les patchs suivants ont été fabriqués :
**(I)** Un patch bicouche selon l'invention renfermant 3 % p/p de trimegestone, grammage de la couche chargée = 59,82 ± 1,77 g/m², grammage de la couche d'adhésion = 29,76 ± 3,67 g/m².
**(II)** Un patch bicouche selon l'invention renfermant 9 % p/p de trimegestone, grammage de la couche chargée = 59,16 ± 2,77 g/m², grammage de la couche d'adhésion = 29,72 ± 3,31 g/m².
**(III)** Un patch tricouche selon l'invention renfermant 9 % p/p de trimegestone.
**(IVa)** Un patch monocouche selon l'invention renfermant 3 % p/p de trimegestone, grammage = 61,1 ± 2,5g/m² (avec un plastifiant : 1 % p/p d'huile de silicone).
**(IVb)** Un patch monocouche selon l'invention, renfermant 3 % p/p de trimegestone, grammage = 59,1 ± 0,9 g/m² (avec un plastifiant : 1 % p/p de Cétiol® S).
**(IVc)** Un patch monocouche selon l'invention renfermant 3 % p/p de trimegestone, grammage = 42,9 ± 3,0 g/m² (sans plastifiant).
**(IVd)** Un patch monocouche selon l'invention renfermant 3 % p/p de trimegestone, grammage = 63,0 ± 3,83 g/m² (sans plastifiant).

### Test d'adhésion :

### Principe :

Le travail d'adhésion instantanée est apprécié par mise en contact d'un patch (surface = 5 cm²) avec une surface en acier (1 cm²) dans des conditions de pression connue (force de compression : 5 N, durée 60 s) et par mesure de l'énergie nécessaire à la séparation des deux surfaces. Cette mesure permet de comparer différentes formules ou de suivre l'évolution de l'adhésion en fonction du temps.

| Type de patch | Travail d'adhésion (N.m) x 10⁻³ |
|---|---|
| (I) bicouche 3 % | 56,4 ± 5,0 |
| (II) bicouche 9 % | 52,1 ± 4,2 |
| (III) tricouche 9 % | 49,4 ± 7,5 |
| (IVa) monocouche 3 % + 1 % huile de silicone | 33,5 ± 1,6 |
| (IVb) monocouche 3 % + 1 % cétiol® S | 31,1 ± 2,0 |
| (IVc) monocouche 3 % (G≈40 g/m²) | 10,2 ± 1,7 |
| (IVd) monocouche 3 % (G≈60 g/m²) | 35,6 ± 2,6 |

En ce qui concerne le patch bicouche ou tricouche, la charge en principe actif contenu dans la matrice chargée ne modifie pas l'adhésion instantanée. La présence d'une ou de deux couches d'adhésif sur la matrice chargée améliore nettement l'adhésion instantanée.

### Flux transcutané ex vivo :

### Principe :

Un fragment de peau est installé sur une cellule pour passage transcutané, type cellule de Franz. Le système transdermique est collé sur la peau, côté stratum corneum. Le principe actif est libéré par le système transdermique, franchit la barrière cutanée et est récupéré dans le milieu récepteur. La quantité de principe actif est alors dosée à intervalle de temps régulier.

### Méthode :

Les fragments de peaux proviennent de chirurgie esthétique (sein ou abdomen).

La peau est conservée à 4°C dans un liquide de survie jusqu'à découpe. Après élimination de la graisse sous-cutanée, la peau est dermatomée à 300 µm d'épaisseur, puis conservée à -30°C jusqu'à utilisation.

La peau est installée sur le compartiment récepteur de la cellule. Il est nécessaire de laisser la peau et le milieu récepteur s'équilibrer pendant 15 heures environ. Le patch est alors placé sur la peau. Le passage transcutané du principe actif est suivi par le dosage de la Trimegestone dans le milieu récepteur.

| Type de patch | Flux (10⁻⁶.g.h⁻¹.cm⁻²) |
|---|---|
| (I) bicouche 3 % | 1,29 ± 0,45 |
| (II) bicouche 9 % | 1,07 ± 0,75 |
| (III) tricouche 9 % | 0,59 ± 0,33 |
| (IVa) monocouche 3 % + 1 % huile de silicone | 0,80 ± 0,54 |
| (IVb) monocouche 3 %+1 % cétiol® S | 0,68 ± 0,22 |
| (IVc) monocouche 3 % (G≈40 g/m²) | 0,34 ± 0,24 |
| (IVd) monocouche 3 % (G≈60 g/m²) | 1,42 ± 0,87 |

- La présence d'une couche ou de deux couches supplémentaire ne modifie pas significativement le flux transcutané.

### TEST SUR L'AFFINITE DE LA TRIMEGESTONE VIS-A-VIS DES RECEPTEURS HORMONAUX

L'affinité de la Trimegestone a été comparée avec d'autres progestomimétiques courants : medroxypropgestérone (MPA), norethistérone (NE), Promegestone (PROM), gestodène (GEST) et levonorgestrel (LNOR). Cette affinité a été étudiée sur les récepteurs hormonaux humains recombinant de la progestérone (hPR), androgène (hAN), glucocorticoide (hGR) et estrogène (hER). Ces récepteurs sont obtenus par surexpression dans un système cellules d'insectes-bacullovirus SF9 selon les méthodes décrites notamment dans la demande de brevet Européenne 0 629 635. Les affinité relatives de liaison (ARL) sont les suivantes :

| Recept. | ARL | | | | | |
|---|---|---|---|---|---|---|
| | Trim. | MPA | NE | PROM | GEST | LNOR |
| hPR | 588 | 298 | 134 | 469 | 868 | 323 |
| hGR | 13 | 58 | 1,4 | 8 | 38 | 7,5 |
| hAN | 2,5 | 36 | 55 | 3 | 71 | 58 |
| hER | < 0,02 | < 0,02 | < 0,15 | < 0,02 | < 0,02 | < 0,02 |

### Etude pharmacocinétique de la Trimegestone

Après application de 4 patchs tricouche de 20 cm² (9 % de Trimegestone) respectivement 4, 3, 4 et 3 jours, la concentration de Trimegestone plasmatique reste stable jusqu'à l'enlèvement du dernier patch avec une valeur moyenne de 1,37 mg/ml. Le flux de Trimegestone a donc une valeur moyenne de 0,4 mg/jour/patch. Aucun changement n'a été observé au niveau des concentrations plasmatique d'estradiol et d'estrone. Les concentrations plasmatiques de FSH et de LH diminuent quand à elles respectivement de 31 et 38 % après 14 jours d'application (effet antigonadotrope de la Trimegestone).

En conclusion, les patchs de Trimegestone selon l'invention appliqués pendant 14 jours (1 tout les trois jours) délivrent un flux constant de Trimegestone avec des concentrations plasmatiques de Trimegestone stables et une bonne tolérance locale.
Figure n° 1 : Structure d'u patch monocouche (20 cm²)
   (a) Film de protection
      (2) Matrice adhésive chargée
   (b) Film protecteur pelable
Figure n° 2 : Structure d'un patch bicouche (20 cm²)
   (a) Film de protection
      (2) Matrice adhésive chargée
      (3) Couche d'adhésion
   (b) Film protecteur pelable
Figure n° 3 : Structure d'un patch tricouche (20 cm²)
   (a) Film de protection
      (1) Couche d'ancrage
      (2) Matrice adhésive chargée
      (3) Couche d'adhésion
   (b) Film protecteur pelable
Figure n°4 :
   (B) Compartiment renfermant l'estradiol (15 cm²)
   (A) Compartiment renfermant la Trimegestone (20 cm²)
      (a') Film de protection
      (a) Film de protection
      (b) Film protecteur pelable
Figure n° 5 :
   (B) Compartiment renfermant l'estradiol
   (A) Compartiment renfermant la Trimegestone
      (a) Film protecteur
      (b) Film protecteur pelable
      (c) Film polyester.

## Revendications

1. Matrice polymère adhésive appliquée à un dispositif destiné à l'administration transdermique d'un progestomimétique **caractérisée en ce que** cette matrice est constituée d'une ou des couches successives suivantes :
- éventuellement une couche (1), dite d'ancrage, constituée d'un polymère silicone,
- une couche (2), constituée d'un polymère silicone chargé en Trimegestone et/ou en un ou plusieurs dérivés pharmaceutiquement acceptables, et éventuellement en plastifiant,
- éventuellement une couche (3), dite d'adhésion, constituée d'un polymère silicone.

2. Matrice polymère adhésive telle que définie à la revendication 1, **caractérisée en ce qu'**elle renferme une quantité de Trimegestone comprise entre 1 % p/p et 10 % p/p.

3. Matrice polymère adhésive selon la revendication 1, **caractérisée en ce que** cette matrice renferme une seule couche (2) constituée d'un polymère silicone chargé en Trimegestone et/ou en un ou plusieurs dérivés pharmaceutiquement acceptables et éventuellement en plastifiant.

4. Matrice polymère adhésive selon la revendication 3, **caractérisée en ce qu'**elle est constituée de 80 à 99 % p/p de polymère silicone ayant un pouvoir adhésif fort chargé en 1 à 10 % p/p de Trimegestone et/ou en un ou plusieurs dérivés pharmaceutiquement acceptables et en 0 à 10 % p/p d'huile de silicone ou de dioctylcyclohexane.

5. Matrice polymère adhésive selon la revendication 4, **caractérisée en ce qu'**elle est constituée de 96 % p/p de polymère silicone ayant un pouvoir adhésif instantané fort chargé en 3 % p/p de Trimegestone et en 1 % p/p d'huile de silicone.

6. Matrice polymère adhésive selon la revendication 1, **caractérisée en ce que** cette matrice renferme deux couches successives :
a) une première couche (2), constituée d'un polymère silicone chargé en Trimegestone et/ou en un ou plusieurs dérivés pharmaceutiquement acceptables,
b) une deuxième couche (3), couche d'adhésion qui sera en contact avec la peau, également constituée d'un polymère silicone.

7. Matrice polymère adhésive selon la revendication 6, **caractérisée en ce que**
a) la première couche, est constituée de 90 à 99 % p/p d'un polymère silicone ayant un pouvoir adhésif fort chargé en 1 à 10 % p/p de Trimegestone et/ou en un ou plusieurs dérivés pharmaceutiquement acceptables,
b) la deuxième couche, est également constituée d'un polymère silicone ayant un pouvoir adhésif fort.

8. Matrice polymère adhésive selon la revendication 7, **caractérisée en ce que**
a) la première couche, est constituée de 97 % p/p d'un polymère silicone ayant un pouvoir adhésif instantané fort, chargé en 3 % p/p de Trimegestone,
b) la deuxième couche, est également constituée d'un polymère silicone ayant un pouvoir adhésif instantané fort.

9. Matrice polymère adhésive selon la revendication 1, **caractérisée en ce que** cette matrice renferme trois couches successives :
a) une première couche (1), dite couche d'ancrage, constituée d'un polymère silicone,
b) une deuxième couche (2) constituée d'un polymère silicone chargé en Trimegestone et/ou en un ou plusieurs dérivés pharmaceutiquement acceptables,
c) et une troisième couche (3), couche d'adhésion, qui doit se trouver sur la peau, constituée elle aussi d'un polymère silicone.

10. Matrice polymère adhésive selon la revendication 9, **caractérisée en ce que**
- la première couche, est constituée d'un polymère silicone ayant un pouvoir adhésif moyen,
- la deuxième couche, est constituée de 90 à 99 % p/p de polymère silicone ayant un pouvoir adhésif moyen chargé en 1 à 10 % p/p de Trimegestone et/ou en un ou plusieurs dérivés pharmaceutiquement acceptables,
- la troisième couche, est constituée d'un polymère silicone ayant un pouvoir adhésif fort.

11. Matrice polymère adhésive selon la revendication 9 ou 10, **caractérisée en ce que**
- la première couche est constituée d'un polymère silicone ayant un pouvoir adhésif instantané moyen,
- la deuxième couche est constituée de 91 % p/p de polymère silicone ayant un pouvoir adhésif instantané moyen chargé en 9 % p/p de Trimegestone,
- la troisième couche est constituée d'un polymère silicone ayant un pouvoir adhésif instantané fort.

12. Dispositif destinée à l'administration transdermique d'un progestomimétique, **caractérisée en ce qu'**il est constitué successivement :
- d'un film de protection (a),
- d'une matrice telle que définie à la revendication 1 ou 2,
- d'un film protecteur pelable (b).

13. Dispositif tel que défini à la revendication 12, **caractérisé en ce que** la matrice est telle que définie à l'une des revendications 3 à 5.

14. Dispositif tel que défini à la revendication 12, **caractérisé en ce que** la matrice est telle que définie à l'une des revendications 6 à 8.

15. Dispositif tel que défini à la revendication 12, **caractérisé en ce que** la matrice est telle que définie à l'une des revendications 9 à 11.

16. Dispositif tel que défini à la revendication 12, **caractérisé en ce que**, en outre, il renferme une matrice chargée en estrogène, ce dispositif étant constitué de deux compartiments (A) et (B).

17. Dispositif tel que défini à la revendication 16, **caractérisé en ce que** le composé estrogène est choisi parmi le 17-béta-estradiol, les esters du 17-béta-estradiol tels que l'estradiol valérate, cyprionate, décanoate et acétate, l'éthynyl estradiol, l'oestrone et un estrogène "d'origine équine" tel que le Premarin® , ou une combinaison de ces composés.

18. Dispositif tel que défini à la revendication 16, **caractérisé en ce que** le composé estrogène est le 17-béta-estradiol.

19. Dispositif tel que défini à la revendication 16, **caractérisé en ce que** les deux compartiments (A) et (B)
- sont supportés par le même film protecteur pelable (b),
- et sont séparés l'un de l'autre par un espace vide ou une barrière de 1 à 10 mm,
- le compartiment (A) renfermant la matrice polymère silicone, chargée en trimegestone et/ou en un ou plusieurs dérivés pharmaceutiquement acceptables, telle que définie à l'une quelconque des revendications 1 à 11,
- le compartiment (B) renfermant une matrice polymère adhésive chargée en estrogène,
- et chacune de ces matrices étant recouverte respectivement d'un film de protection (a) et (a') identique ou différent.

20. Dispositif selon la revendication 19, **caractérisé en ce que** :
- le compartiment (A) renferme une matrice monocouche telle que définie à la revendication 3,
- et le compartiment (B) renferme une matrice monocouche constituée d'un copolymère d'acrylate de 2-éthylhexyle et d'acétate de vinyle, chargé en estradiol, et éventuellement en polymère hydrophile.

21. Dispositif selon la revendication 19 ou 20, **caractérisé en ce que** :
- le compartiment (A) renferme une matrice monocouche, telle que définie à la revendication 4 ou 5,
- et le compartiment (B) renferme une matrice monocouche constituée de 60 à 99 % p/p de copolymère d'acrylate de 2-éthylhexyle (72 %) et d'acétate de vinyle (28 %) chargé en 1 à 10 % p/p d'estradiol et en 0 à 30 % p/p de polyvinylpyrrolidone.

22. Dispositif selon la revendication 19, **caractérisé en ce que** :
- le compartiment (A) renferme une matrice bicouche telle que définie à la revendication 6,
- le compartiment (B) renferme une matrice monocouche constituée d'un copolymère d'acrylate de 2-éthylhexyle et d'acétate de vinyle, chargé en estradiol, et éventuellement en polymère hydrophile.

23. Dispositif selon la revendication 19 ou 22, **caractérisé en ce que** :
- le compartiment (A) renferme une matrice bicouche, telle que définie à la revendication 7 ou 8,
- et le compartiment (B) renferme une matrice monocouche constituée de 60 à 99 % p/p de copolymère d'acrylate de 2-éthylhexyle (72 %) et d'acétate de vinyle (28 %) chargé en 1 à 10 % p/p d'estradiol et en 0 à 30 % p/p de polyvinylpyrrolidone.

24. Dispositif selon la revendication 16, **caractérisé en ce qu'**il est constitué successivement des éléments suivants :
un film de protection (a),
un compartiment (B) constitué d'une matrice polymère adhésive chargée en estrogène tel que l'estradiol,
un film polyester (c) ayant les mêmes dimensions que le compartiment (A) et superposé à celui-ci,
un compartiment (A) constitué de la matrice polymère silicone chargée en Trimegestone et/ou en plusieurs dérivés pharmaceutiquement acceptables, telle que définie ci-dessus, le compartiment (A) étant de dimension inférieure au compartiment (B) telle que la moitié et étant de préférence centré par rapport à ce compartiment (B),
un film de protection-pelable (b).

25. Dispositif selon la revendication 24, **caractérisé en ce que** la matrice renfermant la Trimegestone est une matrice bicouche telle que définie à la revendication 6 ou une matrice tricouche telle que définie à la revendication 9.

26. Procédé de fabrication des dispositifs selon la revendication 12 ou 13, **caractérisé en ce que** :
1 - on dépose préalablement sur un film protecteur pelable (b), une solution de couche adhésive (2) dans l'heptane chargée en Trimegestone et éventuellement en plastifiant, puis on sèche,
2 - on effectue une opération de colaminage de l'ensemble "couche adhésive (2)/film protecteur pelable (b)" sur un film de protection (a),
3 - On découpe les systèmes transdermiques en pastille ayant une surface comprise entre 5 cm² à 50 cm².

27. Procédé de fabrication des dispositifs selon la revendication 12 ou 13, **caractérisé en ce que** :
1 - on dépose préalablement sur un film de protection (a), une solution de couche adhésive (2) dans l'heptane chargée en Trimegestone et éventuellement en plastifiant, puis on sèche,
2 - on effectue une opération de colaminage de l'ensemble "couche adhésive (2)/film de protection (a)" sur un film protecteur pelable (b),
3 - on découpe les systèmes transdermiques en pastille ayant une surface comprise entre 5 cm² à 50 cm².

28. Procédé de fabrication des dispositifs selon la revendication 12 ou 14, **caractérisé en ce que** :
1 - on dépose préalablement
a) sur un film protecteur pelable (b), une solution de couche adhésive (3) dans l'heptane, puis on sèche
b) sur un film protecteur pelable provisoire (b'), une solution de couche adhésive (2) dans l'heptane chargée en Trimegestone, puis on sèche,
2 - on effectue une opération de colaminage de l'ensemble "couché adhésive (2) chargée en Trimegestone/film protecteur pelable provisoire (b')" sur l'ensemble "couche adhésive (3)/film protecteur pelable (b)",
3 - on effectue un pelage du film protecteur provisoire (b')
4 - on effectue une opération de colaminage de l'ensemble "couche adhésive (2)/couche adhésive (3)/film protecteur pelable (b)" sur un film de protection (a)
5 - On découpe les systèmes transdermiques en pastille ayant une surface comprise entre 5 cm² à 50 cm².

29. Procédé de fabrication des dispositifs selon la revendication 12 ou 15, **caractérisé en ce que**
1 -
a) on dépose préalablement sur un film protecteur pelable provisoire (b'), une solution de couche adhésive (1) dans l'heptane, puis on sèche,
b) on dépose préalablement sur un film protecteur pelable provisoire (b"), une solution de couche adhésive (2) dans l'heptane chargée en Trimegestone, puis on sèche,
c) on dépose préalablement sur un film protecteur pelable (b), une solution de couche adhésive (3) dans l'heptane, puis on sèche,
2 - on effectue une opération de colaminage de l'ensemble "couche adhésive (1)/film protecteur pelable provisoire (b')" sur un film de protection (a)
3 - on effectue un pelage du film protecteur provisoire (b')
4 - on effectue une opération de colaminage de l'ensemble "couche adhésive (2) chargée en Trimegestone/film protecteur pelable provisoire (b")" sur l'ensemble "couche adhésive (1)/film de protection (a)",
5 - on effectue le pelage du film protecteur provisoire (b")
6 - on effectue enfin une dernière opération de colaminage du système "couche adhésive (3)/film protecteur pelable (b)" sur l'ensemble "couche chargée (2)/couche adhésive (1)/film de protection (a)".
7 - on découpe les systèmes transdermiques en pastille ayant une surface comprise entre 5 cm² à 50 cm².

30. Procédé de fabrication du dispositif selon la revendication 19, **caractérisé en ce que** :
**Etape I :** pour la fabrication du patch correspondant au compartiment (A)
1 - on enduit la couche polymère adhésive silicone chargée en Trimegestone et éventuellement en un ou plusieurs additifs, sur le film de protection (a),
2 - on évapore le solvant jusqu'à obtention de l'ensemble "matrice chargée en Trimegestone/film protecteur (a)" correspondant au compartiment (A),
3 - on effectue une opération de colaminage de l'ensemble "matrice chargée en Trimegestone/film protecteur (a)" sur un film protecteur pelable (b'),
4 - on découpe un patch de 5 à 50 cm²,
**Etape II :** pour la fabrication du patch correspondant au compartiment (B)
1 - on enduit la couche polymère adhésive chargée en composé estrogène et éventuellement en un ou plusieurs additifs tels qu'un polymère hydrophile, un promoteur d'absorption ou un plastifiant, sur le film de protection (a'),
2 - on évapore le solvant jusqu'à obtention de l'ensemble "matrice chargée en estrogène/film protecteur (a')" correspondant au compartiment (B),
3 - on effectue une opération de colaminage de l'ensemble "matrice chargée en estrogène/film protecteur (a')" sur film protecteur pelable (b"),
4 - on découpe un patch de 5 à 50 cm²,
**Etape III :** pour la formation du "bipatch"
1 - on effectue un pelage du film protecteur pelable (b') du patch obtenu à l'étape I,
2 - puis on transfert l'ensemble "matrice chargée en Trimegestone/film protecteur (a)" sur un film protecteur pelable (b),
3 - on effectue un pelage du film protecteur pelable (b") du patch obtenu à l'étape II,
4 - puis on transfert l'ensemble matrice chargée en "estrogène/film protecteur (a')" sur le film protecteur pelable (b) précédent, en respectant une distance de 1 à 10 mm ou après avoir introduit une barrière entre les deux compartiments (A) et (B).

31. Dispositif selon l'une quelconque des revendications 12 à 16, pour l'utilisation dans un procédé de délivrance, soit de la Trimegestone et/ou un ou plusieurs dérivés pharmaceutiquement acceptable soit de la Trimegestone associée à un estrogène, à un patient par application de la ou des matrices du dispositif à la peau ou à une muqueuse dudit patient.

32. Esters en position 21 de la Trimegestone **caractérisés en ce que** le reste de l'ester renferme 1 atome de carbone ou de 3 à 12 atomes de carbone.

## Patentansprüche

1. Klebende Polymermatrix, die bei einer Vorrichtung angewendet wird, welche zur transdermalen Verabreichung eines Progestomimetikums bestimmt ist, **dadurch gekennzeichnet, daß** die Matrix aus den folgenden aufeinanderfolgenden Schichten oder einer dieser Schichten besteht:
- gegebenenfalls einer sogenannten Verankerungsschicht (1), die aus einem Silicon-Polymer besteht;
- einer Schicht (2), die aus einem Silicon-Polymer besteht, das Trimegeston und/oder ein oder mehrere pharmazeutisch annehmbare Derivate und gegebenenfalls einen Weichmacher enthält;
- gegebenenfalls einer sogenannten Adhäsionsschicht, die aus einem Silicon-Polymer besteht.

2. Klebende Polymermatrix nach Anspruch 1, **dadurch gekennzeichnet, daß** sie eine Trimegestonmenge zwischen 1 Gew.-% und 10 Gew.-% umfaßt.

3. Klebende Polymermatrix nach Anspruch 1, **dadurch gekennzeichnet, daß** die Matrix eine einzige Schicht (2) umfaßt, die aus einem Silicon-Polymer besteht, das Trimegeston und/oder ein oder mehrere pharmazeutisch annehmbare Derivate und gegebenenfalls einen Weichmacher enthält.

4. Klebende Polymermatrix nach Anspruch 3, **dadurch gekennzeichnet, daß** sie aus 80 bis 99 Gew.-% Silicon-Polymer, das eine starke Klebekraft hat und das 1 bis 10 Gew.-% Trimegeston und/oder ein oder mehrere pharmazeutisch annehmbare Derivate und 0 bis 10 Gew.-% Siliconöl oder Dioctylcyclohexan enthält, besteht.

5. Klebende Polymermatrix nach Anspruch 4, **dadurch gekennzeichnet, daß** sie aus 96 Gew.-% Silicon-Polymer, das eine sofort wirkende starke Klebekraft hat und das 3 Gew.-% Trimegeston und 1 Gew.-% Siliconöl enthält, besteht.

6. Klebende Polymermatrix nach Anspruch 1, **dadurch gekennzeichnet, daß** die Matrix zwei aufeinanderfolgende Schichten umfaßt:
a) eine erste Schicht (2), die aus einem Silicon-Polymer, das Trimegeston und ein oder mehrere pharmazeutisch annehmbare Derivate enthält, besteht;
b) eine zweite Schicht (3), eine Adhäsionsschicht, die mit der Haut in Kontakt sein wird und die ebenfalls aus einem Silicon-Polymer besteht.

7. Klebende Polymermatrix nach Anspruch 6, **dadurch gekennzeichnet, daß**
a) die erste Schicht aus 90 bis 99 Gew.-% Silicon-Polymer, das eine starke Klebekraft hat und 1 bis 10 Gew.-% Trimegeston und/oder ein oder mehrere pharmazeutisch annehmbare Derivate enthält, besteht;
b) die zweite Schicht ebenfalls aus einem Silicon-Polymer, das eine starke Klebekraft hat, besteht.

8. Klebende Polymermatrix nach Anspruch 7, **dadurch gekennzeichnet, daß**
a) die erste Schicht aus 97 Gew.-% Silicon-Polymer, das eine sofort wirkende starke Klebekraft hat und das 3 Gew.-% Trimegeston enthält, besteht;
b) die zweite Schicht ebenfalls aus einem Silicon-Polymer, das eine sofort wirkende starke Klebekraft hat, besteht.

9. Klebende Polymermatrix nach Anspruch 1, **dadurch gekennzeichnet, daß** die Matrix drei aufeinanderfolgende Schichten umfaßt:
a) eine erste Schicht (1), die sogenannte Verankerungsschicht, die aus einem Silicon-Polymer besteht;
b) eine zweite Schicht (2), die aus einem Silicon-Polymer besteht, das Trimegeston und/oder ein oder mehrere pharmazeutisch annehmbare Derivate enthält;
c) und eine dritte Schicht (3), die sogenannte Adhäsionsschicht, die sich auf der Haut befinden wird und die ebenfalls aus einem Silicon-Polymer besteht.

10. Klebende Polymermatrix nach Anspruch 9, **dadurch gekennzeichnet, daß**
- die erste Schicht aus einem Silicon-Polymer besteht, das eine mittlere Klebekraft hat;
- die zweite Schicht aus 90 bis 99 Gew.-% Silicon-Polymer, das eine mittlere Klebekraft hat und das 1 bis 10 Gew.-% Trimegeston und/oder ein oder mehrere pharmazeutisch annehmbare Derivate enthält, besteht;
- die dritte Schicht aus einem Silicon-Polymer, das eine starke Klebekraft hat, besteht.

11. Klebende Polymermatrix nach Anspruch 9 oder 10, **dadurch gekennzeichnet, daß**
- die erste Schicht aus einem Silicon-Polymer, das eine sofort wirkende mittlere Klebekraft hat, besteht;
- die zweite Schicht aus 91 Gew.-% Silicon-Polymer, das eine sofort wirkende mittlere Klebekraft hat und 9 Gew.-% Trimegeston enthält, besteht;
- die dritte Schicht aus einem Silicon-Polymer, das eine sofort wirkende starke Klebekraft hat, besteht.

12. Vorrichtung, die zur transdermalen Verabreichung eines Progestomimetikum bestimmt ist, **dadurch gekennzeichnet, daß** sie aufeinanderfolgend aus
- einem Schutzfilm (a),
- einer Matrix, wie sie in Anspruch 1 oder 2 definiert ist,
- einem abziehbaren Schutzfilm (b) besteht.

13. Vorrichtung nach Anspruch 12, **dadurch gekennzeichnet, daß** die Matrix eine ist, wie sie in einem der Ansprüche 3 bis 5 definiert ist.

14. Vorrichtung nach Anspruch 12, **dadurch gekennzeichnet, daß** die Matrix eine ist, wie sie in einem der Ansprüche 6 bis 8 definiert ist.

15. Vorrichtung nach Anspruch 12, **dadurch gekennzeichnet, daß** die Matrix eine ist, wie sie in einem der Ansprüche 9 bis 11 definiert ist.

16. Vorrichtung nach Anspruch 12, **dadurch gekennzeichnet, daß** sie außerdem eine Matrix umfaßt, die Östrogen enthält, wobei diese Vorrichtung aus zwei Bereichen (A) und (B) besteht.

17. Vorrichtung nach Anspruch 16, **dadurch gekennzeichnet, daß** die Östrogen-Verbindung aus 17-β-Estradiol, den Estern von 17-β-Estradiol wie z.B. Estradiolvalerat, - cyprionat, -decanoat und -acetat, Ethinylestradiol, Östron und einem Östrogen "aus Pferden" wie auch Premarin® oder einer Kombination dieser Verbindungen ausgewählt ist.

18. Vorrichtung nach Anspruch 16, **dadurch gekennzeichnet, daß** die Östrogen-Verbindung 17-β-Estradiol ist.

19. Vorrichtung nach Anspruch 16, **dadurch gekennzeichnet, daß** die zwei Bereiche (A) und (B)
- durch denselben abziehbaren Schutzfilm (b) getragen werden,
- und durch einen leeren Raum oder eine Barriere von 1 bis 10 mm voneinander getrennt sind,
- der Bereich (A) die Silicon-Polymermatrix, die Trimegeston und/oder ein oder mehrere pharmazeutisch annehmbare Derivate enthält, nach einem der Ansprüche 1 bis 11 umfaßt,
- der Bereich (B) eine klebende Polymermatrix, die Östrogen enthält, umfaßt,
- und jede dieser Matrizes von einem Schutzfilm (a) bzw. (a'), die gleich oder unterschiedlich sind, überzogen ist.

20. Vorrichtung nach Anspruch 19, **dadurch gekennzeichnet, daß**
- der Bereich (A) eine einschichtige Matrix, wie sie in Anspruch 3 definiert ist, umfaßt,
- und der Bereich (B) eine einschichtige Matrix umfaßt, die aus einem Copolymer aus 2-Ethylhexylacrylat und Vinylacetat, das Estradiol und gegebenenfalls ein hydrophiles Polymer enthält, besteht.

21. Vorrichtung nach Anspruch 19 oder 20, **dadurch gekennzeichnet, daß**
- der Bereich (A) eine einschichtige Matrix, wie sie in Anspruch 4 oder 5 definiert ist, umfaßt,
- und der Bereich (B) eine einschichtige Matrix umfaßt, die aus 60 bis 99 Gew.-% Copolymer aus 2-Ethylhexylacrylat (72 %) und Vinylacetat (28 %), das 1 bis 10 Gew.-% Estradiol und 0 bis 30 Gew.-% Polyvinylpyrrolidon enthält, besteht.

22. Einrichtung nach Anspruch 19, **dadurch gekennzeichnet daß**
- der Bereich (A) eine zweischichtige Matrix, wie sie in Anspruch 6 definiert ist, umfaßt,
- der Bereich (B) eine einschichtige Matrix umfaßt, die aus einem Copolymer aus 2-Ethylhexyl crylat und Vinylacetat, das Estradiol und gegebenenfalls hydrophiles Polymer enthält, besteht.

23. Vorrichtung nach Anspruch 19 oder 22, **dadurch gekennzeichnet, daß**
- der Bereich (A) eine zweischichtige Matrix, wie sie in Anspruch 7 oder 8 definiert ist, umfaßt,
- und der Bereich (B) eine einschichtige Matrix umfaßt, die aus 60 bis 99 Gew.-% Copolymer aus 2-Ethylhexylacrylat (72 %) und Vinylacetat (28 %), das 1 bis 10 Gew.-% Estradiol und 0 bis 30 Gew.-% Polyvinylpyrrolidon enthält, besteht.

24. Vorrichtung nach Anspruch 16, **dadurch gekennzeichnet, daß** sie aufeinanderfolgend aus den folgenden Elementen besteht:
- einem Schutzfilm (a),
- einem Bereich (B), der aus einer klebenden Polymermatrix, die Östrogen wie z.B. Estradiol enthält, besteht;
- einem Polyesterfilm (c), der dieselben Abmessungen wie der Bereich (A) hat und über diesem angeordnet ist,
- einem Bereich (A), der aus der oben definierten Silicon-Polymermatrix, die Trimegeston und/oder mehrere pharmazeutisch annehmbare Derivate enthält, wie sie oben definiert ist, besteht, wobei der Bereich (A) eine kleinere Abmessung als der Bereich (E) hat, z.B. die Hälfte, und vorzugsweise bezüglich des Bereichs (B) zentriert angeordnet ist,
- einem abziehbaren Schutzfilm (b).

25. Vorrichtung nach Anspruch 24, **dadurch gekennzeichnet, daß** die Matrix, die das Trimegaston umfaßt, eine zweischichtige.Matrix, wie sie in Anspruch 6 definiert ist, oder eine dreischichtige Matrix, wie sie in Anspruch 9 definiert ist, ist.

26. Verfahren zur Herstellung von Vorrichtungen nach Anspruch 12 oder 13, **dadurch gekennzeichnet, daß**
1 - man vorab auf einen abziehbaren Schutzfilm (b) eine Lösung für eine Klebeschicht (2) in Heptan, die Trimegeston und gegebenenfalls Weichmacher enthält, aufträgt, dann trocknet,
2 - man einen Vorgang des Klebens der Einheit "Klebeschicht (2)/abziehbarer Schutzfilm (b)" auf einen Schutzfilm (a) durchführt,
3 - man die transdermalen Systeme in Stücke mit einer Oberfläche von 5 cm² bis 50 cm² schneidet.

27. Verfahren zur Herstellung der Vorrichtungen nach Anspruch 12 oder 13, **dadurch gekennzeichnet, daß**
1 - man vorab auf einen Schutzfilm (a) eine Lösung für eine Klebeschicht (2) in Heptan, die Trimegeston und gegebenenfalls Weichmacher enthält, aufträgt, dann trocknet,
2 - man einen Vorgang des Klebens der Einheit "Klebeschicht (2)/Schutzfilm (a)" auf einen abziehbaren Schutzfilm (b) durchführt,
3 - man die transdermalen Systeme in Stücke mit einer Oberfläche von 5 cm² bis 50 cm² schneidet.

28. Verfahren zur Herstellung der Vorrichtungen gemäß Anspruch 12 oder 14, **dadurch gekennzeichnet, daß**
1 - man vorab
a) auf einen abziehbaren Schutzfilm (b) eine Lösung für die Klebeschicht (3) in Heptan auf einen abziehbaren Schutzfilm (b) aufträgt, dann trocknet,
b) auf einen provisorischen abziehbaren Schutzfilm (b') eine Lösung für eine Klebeschicht (2) in Heptan, die Trimegeston enthält, aufträgt, dann trocknet,
2 - man einen Vorgang des Klebens der Einheit "Klebeschicht (2), die Trimegeston enthält/provisorischer abziehbarer Schutzfilm (b')" auf die Einheit "Klebeschicht (3)/abziehbarer Schutzfilm (b)" durchführt,
3 - man ein Abziehen des provisorischen Schutzfilms (b') durchführt,
4 - man einen Vorgang des Klebens der Einheit "Klebeschicht (2)/Klebeschicht (3)/abziehbarer Schutzfilm (b')" auf einen Schutzfilm (a) durchführt,
5 - man die transdermalen Systeme in Stücke mit einer Oberfläche von 5 cm² bis 50 cm² schneidet.

29. Verfahren zur Herstellung von Vorrichtungen nach Anspruch 12 oder 15, **dadurch gekennzeichnet, daß**
1 - a) man vorab auf einen provisorischen abziehbaren Schutzfilm (b') eine Lösung für eine Klebeschicht (1) in Heptan aufträgt, man dann trocknet,
b) man vorab auf einen provisorischen abziehbaren Schutzfilm (b") eine Lösung für eine Klebeschicht (2) in Heptan, die Trimegeston enthält, aufträgt, man dann trocknet,
c) man vorab auf einen abziehbaren Schutzfilm (b) eine Lösung für eine Klebeschicht (3) in Heptan aufträgt, man dann trocknet,
2 - man einen Vorgang des Klebens der Einheit "Klebeschicht (1)/provisorischer abziehbarer Schutzfilm (b')" auf einen Schutzfilm (a) durchführt,
3 - man ein Abziehen des provisorischen Schutzfilms (b') durchführt,
4 - man einen Vorgang des Klebens der Einheit "Klebeschicht (2), die Trimegeston enthält/provisorischer abziehbarer Schutzfilm (b")" auf die Einheit "Klebeschicht (1)/Schutzfilm (a)" durchführt,
5 - man ein Abziehen des provisorischen Schutzfilms (b") durchführt,
6 - man schließlich einen letzten Vorgang des Klebens des Systems "Klebeschicht (3)/abziehbarer Schutzfilm (b)" auf die Einheit "beladene Schicht (2)/Klebeschicht (1)/Schutzfilm (a)" durchführt,
7 - man die transdermalen Systeme in Stücke mit einer Oberfläche von 5 cm² bis 50 cm² schneidet.

30. Verfahren zur Herstellung einer Vorrichtung nach Anspruch 19, **gekennzeichnet durch**:
Stufe I: für die Herstellung des Patches, das Bereich (A) entspricht.
1 - man trägt die klebende Silicon-Polymerschicht, die Trimegeston und gegebenenfalls ein oder mehrere Additive enthält, auf den Schutzfilm (a) auf,
2 - man verdampft das Lösungsmittel bis zum Erhalt der Einheit "Matrix, die Trimegeston enthält/Schutzfilm (a)", die dem Bereich (A) entspricht,
3 - man führt einen Vorgang des Klebens der Einheit "Matrix, die Trimegeston enthält/Schutzfilm (a)" auf einen abziebaren Schutzfilm (b') **durch**,
4 - man schneidet ein Patch mit 5 bis 50 cm² zu;
Stufe II: zur Herstellung des Patchs, das Bereich (B) entspricht.
1 - man trägt die klebende Polymerschicht, die eine Östrogen-Verbindung und gegebenenfalls ein oder mehrere Additive wie z.B. ein hydrophiles Polymer, einen Absorptionspromotor oder einen Weichmacher enthält, auf den Schutzfilm (a') auf,
2 - man verdampft das Lösungsmittel bis zum Erhalt der Einheit "Matrix, die ein Östrogen enthält/Schutzfilm (a')", die dem Bereich (B) entspricht,
3 - man führt einen Vorgang des Klebens der Einheit "Matrix, die ein Östrogen enthält/Schutzfilm (a')" auf einen abziehbaren Schutzfilm (b") **durch**,
4 - man schneidet ein Patch mit 5 bis 50 cm² zu;
Stufe III: zur Bildung des "Bipatch"
1 - man führt ein Abziehen des abziehbaren Schutzfilms (b') des in Stufe I erhaltenen Patchs **durch**,
2 - dann transferiert man die Einheit "Matrix, die Trimegeston enthält/Schutzfilm (a)" auf einen abziehbaren Schutzfilm (b),
3 - man führt ein Abziehen des abziehbaren Schutzfilms (b") des in Stufe II erhaltenen Patchs **durch**,
4 - dann transferiert man die Einheit "Matrix, die ein Östrogen enthält/Schutzfilm (a')" auf den vorher genannten abziehbaren Schutzfilm (b) unter Wahrung eines Abstandes von 1 bis 10 mm oder nach Einführung einer Barriere zwischen den beiden Bereichen (A) und (B).

31. Vorrichtung nach einem der Ansprüche 12 bis 16 zur Verwendung in einem Verfahren zur Zuführung von Trimegeston und/oder einem oder mehreren pharmazeutisch annehmbaren Derivaten oder von Trimegeston in Assoziation mit einem Östrogen bei einem Patienten durch Aufbringen der Matrix (der Matrizes) der Vorrichtung auf die Haut oder eine Schleimhaut des Patienten.

32. Ester in Position 21 des Trimegestons, **dadurch gekennzeichnet, daß** der Ester-Rest 1 Kohlenstoffatom oder 3 bis 12 Kohlenstoffatome umfaßt.

## Claims

1. Adhesive polymer matrix applied to a device intended for the transdermal administration of a progestomimetic **characterized in that** this matrix is constituted by one or more of the following successive layers:
- optionally a layer (1), called an anchoring layer, constituted by a silicone polymer,
- a layer (2), constituted by a silicone polymer loaded with Trimegestone and/or one or more pharmaceutically acceptable derivatives, and optionally with plasticizer,
- optionally a layer (3), called an adhesion layer, constituted by a silicone polymer.

2. Adhesive polymer matrix as defined in claim 1, **characterized in that** it contains a quantity of Trimegestone comprised between 1 % w/w and 10 % w/w.

3. Adhesive polymer matrix according to claim 1, **characterized in that** this matrix contains a single layer (2) constituted by a silicone polymer loaded with Trimegestone and/or one or more pharmaceutically acceptable derivatives and optionally with plasticizer.

4. Adhesive polymer matrix according to claim 3, **characterized in that** it is constituted by 80 to 99 % w/w of silicone polymer having a strong adhesive power loaded with 1 to 10 % w/w of Trimegestone and/or in one or more pharmaceutically acceptable derivatives and in 0 to 10 % w/w of silicone oil or dioctylcyclohexane.

5. Adhesive polymer matrix according to claim 4, **characterized in that** it is constituted by 96 % w/w of silicone polymer having a strong instantaneous adhesive power loaded with 3 % w/w of Trimegestone and in 1 % w/w of silicone oil.

6. Adhesive polymer matrix according to claim 1, **characterized in that** this matrix contains two successive layers:
a) a first layer (2), constituted by a silicone polymer loaded with Trimegestone and/or one or more pharmaceutically acceptable derivatives,
b) a second layer (3), an adhesion layer which will be in contact with the skin, also constituted by a silicone polymer.

7. Adhesive polymer matrix according to claim 6, **characterized in that**
a) the first layer, is constituted by 90 to 99 % w/w of a silicone polymer having a strong adhesive power loaded with 1 to 10 % w/w of Trimegestone and/or one or more pharmaceutically acceptable derivatives,
b) the second layer, is also constituted by a silicone polymer having a strong adhesive power.

8. Adhesive polymer matrix according to claim 7, **characterized in that**
a) the first layer, is constituted by 97 % w/w of a silicone polymer having a strong instantaneous adhesive power, loaded with 3 % w/w of Trimegestone,
b) the second layer, is also constituted by a silicone polymer having a strong instantaneous adhesive power.

9. Adhesive polymer matrix according to claim 1, **characterised in that** this matrix contains three successive layers:
a) a first layer (1), called an anchoring layer, constituted by a silicone polymer,
b) a second layer (2) constituted by a silicone polymer loaded with Trimegestone and/or one or more pharmaceutically acceptable derivatives,
c) and a third layer (3), an adhesion layer, which must be placed on the skin, also constituted by a silicone polymer.

10. Adhesive polymer matrix according to claim 9, **characterized in that**
- the first layer, is constituted by a silicone polymer having an average adhesive power,
- the second layer, is constituted by 90 to 99 % w/w of silicone polymer having an average adhesive power loaded with 1 to 10 % w/w of Trimegestone and/or one or more pharmaceutically acceptable derivatives,
- the third layer, is constituted by a silicone polymer having a strong adhesive power.

11. Adhesive polymer matrix according to claim 9 or 10, **characterized in that**
- the first layer is constituted by a silicone polymer having an average instantaneous adhesive power,
- the second layer is constituted by 91 % w/w of silicone polymer having an average instantaneous adhesive power loaded with 9 % w/w of Trimegestone,
- the third layer is constituted by a silicone polymer having a strong instantaneous adhesive power.

12. Device intended for the transdermal administration of a progestomimetic, **characterized in that** it is constituted successively:
- by a protective film (a),
- by a matrix as defined in claim 1 or 2,
- by a peelable protective film (b).

13. Device as defined in claim 12, **characterized in that** the matrix is as defined in one of claims 3 to 5.

14. Device as defined in claim 12, **characterized in that** the matrix is as defined in one of claims 6 to 8.

15. Device as defined in claim 12, **characterized in that** the matrix is as defined in one of claims 9 to 11.

16. Device as defined in claim 12, **characterized in that**, it moreover contains a matrix loaded with estrogen, this device being constituted by two compartments (A) and (B).

17. Device as defined in claim 16, **characterized in that** the estrogen compound is chosen from 17-beta-estradiol, the esters of 17-beta-estradiol such estradiol valerate, cyprionate, decanoate and acetate, ethynyl estradiol, estrone and an estrogen "of equine origin" such as Premarin® , or a combination of these compounds.

18. Device as defined in claim 16, **characterized in that** the estrogen compounds are 17-beta-estradiol.

19. Device as defined in claim 16, **characterized in that** the two compartments (A) and (B)
- are supported by the same peelable protective film (b),
- and are separated from one another by an empty space or a barrier of 1 to 10 mm,
- compartment (A) containing the silicone polymer matrix, loaded with trimegestone and/or one or more pharmaceutically acceptable derivatives, as defined in any one of claims 1 to 11,
- compartment (B) containing an adhesive polymer matrix loaded with estrogen,
- and each of these matrices being respectively covered with a protective film (a) and (a') identical or different.

20. Device according to claim 19, **characterized in that**:
- compartment (A) contains a single layer matrix as defined in claim 3,
- and compartment (B) contains a single layer matrix constituted by a 2-ethylhexyl acrylate and vinyl acetate copolymer, loaded with estradiol, and optionally with hydrophilic polymer.

21. Device according to claim 19 or 20, **characterized in that**:
- compartment (A) contains a single layer matrix, as defined in claim 4 or 5,
- and compartment (B) contains a single layer matrix constituted by 60 to 99 % w/w of 2-ethylhexyl acrylate (72 %) and vinyl acetate (28 %) copolymer loaded with 1 to 10 % w/w of estradiol and 0 to 30 % w/w of polyvinylpyrrolidone.

22. Device according to claim 19, **characterized in that**:
- compartment (A) contains a double layer matrix as defined in claim 6,
- compartment (B) contains a single layer matrix constituted by a 2-ethylhexyl acrylate and vinyl acetate copolymer, loaded with estradiol, and optionally with hydrophilic polymer.

23. Device according to claim 19 or 22, **characterized in that**:
- compartment (A) contains a double layer matrix, as defined in claim 7 or 8,
- and compartment (B) contains a single layer matrix constituted by 60 to 99 % w/w of 2-ethylhexyl acrylate (72 %) and vinyl acetate (28 %) copolymer loaded with 1 to 10 % w/w of estradiol and 0 to 30 % w/w of polyvinylpyrrolidone.

24. Device according to claim 16, **characterized in that** it is constituted successively of the following elements:
a protective film (a),
a compartment (B) constituted by an adhesive polymer matrix loaded with estrogen such as estradiol,
a polyester film (c) having the same dimensions as compartment (A) and superposed on it,
a compartment (A) constituted by the silicone polymer matrix loaded with Trimegestone and/or several pharmaceutically acceptable derivatives, as defined above, compartment (A) being of smaller dimensions than compartment (B) such as half its size and being preferably cantered in relation to this compartment (B),
a peelable protective film (b).

25. Device according to claim 24, **characterized in that** the matrix containing Trimegestone is a double layer matrix as defined in claim 6 or a triple layer matrix as defined in claim 9.

26. Manufacturing process for the devices according to claim 12 or 13, **characterized in that**:
1 - an adhesive layer solution (2) in heptane loaded with Trimegestone and optionally with plasticizer is deposited beforehand on a peelable protective film (b), then dried,
2 - co-lamination of the "adhesive layer (2)/peelable protective film (b)" on a protective film (a) is carried out,
3 - the transdermal systems are cut into patches with a surface comprised between 5 cm² to 50 cm².

27. Manufacturing process for the devices according to claim 12 or 13, **characterized in that**:
1 - an adhesive layer solution (2) in heptane loaded with Trimegestone and optionally with plasticizer is deposited beforehand on a protective film (a), then dried,
2 - a co-lamination operation of the "adhesive layer (2)/protective film (a)" assembly on a peelable protective film (b) is carried out,
3 - the transdermal systems are cut into patches having a surface comprised between 5 cm² to 50 cm².

28. Manufacturing process for the devices according to claim 12 or 14, **characterized in that**:
1 -
a) an adhesive layer solution (3) in heptane is deposited beforehand on a peelable protective film (b), then dried
b) an adhesive layer solution (2) in heptane loaded with Trimegestone is deposited beforehand on a temporary peelable protective film (b'), then dried,
2 - a co-lamination operation of the "adhesive layer (2) loaded with Trimegestone/temporary peelable protective film (b')" assembly on the "adhesive layer is carried out (3)/peelable protective film (b)" assembly,
3 - the temporary protective film (b') is peeled off
4 - a co-lamination operation of the "adhesive layer (2)/adhesive layer (3)/peelable protective film (b)" assembly on a protective film (a) is carried out
5 - the transdermal systems are cut into patches with a surface comprised between 5 cm² to 50 cm².

29. Manufacturing process for the devices according to claim 12 or 15, **characterized in that**
1 -
a) an adhesive layer solution (1) in heptane is deposited beforehand on a temporary peelable protective film (b'), then dried,
b) an adhesive layer solution (2) in heptane loaded with Trimegestone is deposited beforehand on a temporary peelable protective film (b"), then dried,
c) an adhesive layer solution (3) in heptane is deposited beforehand on a peelable protective film (b, then dried,
2 - a co-lamination operation of the "adhesive layer (1)/temporary peelable protective film (b')" assembly on a protective film (a) is carried out
3 - the temporary protective film (b') is peeled off
4 - a co-lamination operation of the "adhesive layer (2) loaded with Trimegestone/temporary peelable protective film (b")" assembly on the "adhesive layer (1)/protective film (a)" assembly is carried out,
5 - the temporary protective film (b") is peeled off
6 - finally a last co-lamination operation of the "adhesive layer (3)/peelable protective film (b)" system onto the "layer loaded (2)/adhesive layer (1)/protective film (a)" assembly is carried out.
7 - the transdermal systems are cut into patches with a surface comprised between 5 cm² to 50 cm².

30. Manufacturing process for the device according to claim 19, **characterized in that**:
**Stage I:** for the manufacture of the patch corresponding to compartment (A)
1 - the protective film (a), is coated with the adhesive silicone polymer layer loaded with Trimegestone and optionally with one or more additives,
2 - the solvent is evaporated off until the "matrix loaded with Trimegestone/protective film (a)" assembly corresponding to compartment (A) is obtained,
3 - a co-lamination operation -rolling of the "matrix loaded with Trimegestone/protective film (a)" assembly onto a peelable protective film (b') is carried out,
4 - a patch of 5 to 50 cm² is cut out,
**Stage II:** for the manufacture of the patch corresponding to compartment (B)
1 - the protective film (a') is coated with the polymer adhesive layer loaded with estrogen compounds and optionally with one or more additives such as a hydrophilic polymer, an absorption promoter or a plasticizer,
2 - the solvent is evaporated off until the "matrix loaded with estrogen/protective film (a')" assembly corresponding to compartment (B),
3 - a co-lamination operation of the "matrix loaded with estrogen/protective film (a')" assembly onto the peelable protective film (b") is carried out,
4 - a patch of 5 to 50 cm² is cut out,
**Stage III:** for the formation of the "bipatch"
1 - the peelable protective film (b') is peeled off from the patch obtained in the stage I ,
2 - then the "matrix loaded with Trimegestone/protective film (a)" assembly is transferred onto a peelable protective film (b),
3 - the peelable protective film (b") is peeled off from the patch obtained in stage II,
4 - then the matrix loaded with "estrogen/protective film (a')" assembly is transferred onto the previous peelable protective film (b), whilst respecting a distance of 1 to 10 mm or after having introduced a barrier between the two compartments (A) and (B).

31. Device according to any one of claims 12 to 16, for the use in a process of delivery, either of Trimegestone and/or one or more pharmaceutically acceptable derivatives or of Trimegestone combined with estrogen, to a patient by application of the matrix/matrices of the device onto the skin or to a mucous membrane of said patient.

32. Esters in position 21 of the Trimegestone **characterised in that** the remainder of the ester contains 1 carbon atom or 3 to 12 carbon atoms.
